# EUROPEAN PATENT SPECIFICATION

(11) **EP 1 641 771 B1**
(45) Date of publication and mention of the grant of the patent: **15.10.2008**
(21) Application number: 04742991.5
(22) Date of filing: 18.06.2004
(51) Int. Cl.: C07D 295/22, A61K 31/496, A61P 37/08

(54) **HYDROXAMATE SULFONAMIDES AS CD23 SHEDDING INHIBITORS**
HYDROXAMATSULFONAMIDE ALS INHIBITOREN VON CD23-SHEDDING
SULFONAMIDES D'HYDROXAMATE SERVANT D'INHIBITEURS D'ELIMINATION DU VIRUS CD23

(30) Priority: 19.06.2003 GB 0314244; 05.11.2003 GB 0325834
(43) Date of publication of application: 05.04.2006
(73) Proprietor: UCB Pharma, S.A., 1070 Brussels (BE)
(72) Inventor: OWEN, David, Alan CELLTECH R & D LIMITED, Slough, Berkshire SL1 3WE (GB); WATSON, Robert, John CELLTECH R & D LIMITED, Slough, Berkshire SL1 3WE (GB); ALLEN, Daniel, Rees CELLTECH R & D LIMITED, Slough, Berkshire SL1 3WE (GB); SHARPE, Andrew CELLTECH R & D LIMITED, Slough, Berkshire SL1 3WE (GB); DYKE, Hazel, Joan CELLTECH R & D LIMITED, Slough, Berkshire SL1 3WE (GB)
(74) Representative: Lechien, Monique
(86) International application number: PCT/GB2004/002638
(87) International publication number: WO 2004/113312

(56) References cited:
- WO-A-00/12478
- WO-A-01/62715
- WO-A-01/62751
- WO-A-01/85721
- MAYER R J ET AL: "CD23 shedding: requirements for substrate recognition and inhibition by dipeptide hydroxamic acids" INFLAMMATION RESEARCH, vol. 51, 2002, pages 085-090, XP002296705

## Description

### Field of the Invention

This invention relates to a series of novel hydroxamate sulfonamides and their derivatives, to processes for their preparation, to pharmaceutical compositions containing them and to their use in medicine.

### Background of the Invention

CD23, which is also known as the low affinity receptor for immunoglobulin (Ig)E (FcεRII), is a type II integral protein expressed on a variety of haematopoietic and structural cells. In humans CD23 is a Ca²⁺ dependent C-type lectin of 45 kDa and exists under two forms, CD23a and CD23b (Clin. and Exp. Allergy, 2000, 30, pp. 602-605). Both types are found on B-cells; CD23a is expressed constitutively and CD23b is induced in particular by IL-4. The b isoform is also found on non-B-cells such as T-cells, Langerhans cells, monocytes, macrophages, platelets and eosinophils.

CD23 is not only an IgE receptor, but also a membrane-bound precursor of soluble molecules that still bind IgE (sCD23 or IgE-binding factors) (Sarfati, M. et al., Immunol. Res., 1992, 11, pp. 260-272). sCD23 of molecular weights 37, 33, 29, 25 and 17 kDa arise by an autocatalytic cleavage process involving a metalloprotease cleavage of membrane-bound CD23 (Marolewski, A. et al., Biochem. J., 1998, 333, pp. 573-579).

Membrane-bound CD23 is a multifunctional molecule, which may exert different functions according to the cell type on which it is expressed, ranging from cellular adhesion, antigen presentation, growth and differentiation of Band T-cells, rescue from apoptosis, release of cytotoxic mediators and regulation of IgE synthesis (Bonnefoy, J. et al., Int. Rev. Immunol., 1997, 16, pp. 113-128). It has been postulated that CD23 is overexpressed in several pathologic conditions such as allergic, autoimmune, and parasite diseases, and B-cell lymphoproliferative diseases, such as chronic lymphocytic leukemia.

There is increasing evidence that sCD23 fragments may exert several effects, either alone or in conjunction with other cytokines, on a large variety of haematopoietic cells. These effects include the regulation of IgE synthesis, promotion of B- and T-cell proliferation, and inhibition of monocyte migration, and in synergy with interleukin 1 (IL1) sCD23 fragments may be implicated in the differentiation of early thymocytes, myeloid cell precursors and some germinal centre B-cells.

In particular the three higher molecular weight sCD23 fragments (37, 33 and 29 kDa) have multifunctional cytokine properties which appear to play a major role in IgE production. The excessive formation of sCD23 has been implicated in the overproduction of IgE, which is the hallmark of allergic diseases such as extrinsic asthma, rhinitis, allergic conjunctivitis, eczema, atopic dermatitis and anaphylaxis (Sutton and Gould, Nature, 1993, 366, pp. 421-428). Elevated levels of sCD23 have also been observed in the synovial fluids of patients with rheumatoid arthritis (Chomarat, P. et al., Arthritis and Rheumatism, 1993, 36, pp. 234-242).

It has been shown that crosslinking CD23 at the cell surface by IgE delivers a negative feedback for IgE production and inhibits the release of sCD23. However, sCD23 fragments larger than 25 kDa that retain part of the stalk region may promote IgE production by at least two mechanisms: 1) sCD23 directly stimulates IgE production possibly through CD21 triggering; 2) sCD23 fragments are capable of trapping IgE in the medium and thus may prevent negative feedback through membrane-bound CD23. Thus, compounds which have the ability to inhibit the formation of sCD23 should have twofold actions of: 1) inhibiting the immunostimulatory activities of the higher molecular weight soluble fragments; 2) enhancing negative feedback inhibition of IgE synthesis by maintaining levels of CD23 on the surface of B-cells. In addition, inhibition of CD23 cleavage should lessen sCD23-induced monocyte activation and mediator formation, thereby reducing the inflammatory response.

Until recently the therapeutic approach to modulating allergic responses has been focussed on the mediators thought to cause the response rather than addressing directly the control of IgE production (Christie, G. et al., Eur. J. Immunol., 1997, 27, pp. 3228-3235). One proposed approach for a therapeutically relevant control point in the regulation of IgE synthesis is the regulation of CD23 processing to sCD23.

International patent application no. WO 01/85721 discloses certain cyclised N-sulfonyl hydroxamic acid derivatives as inhibitors of CD 23.

International patent application no. WO 01/62715 discloses certain compounds of formula (1): 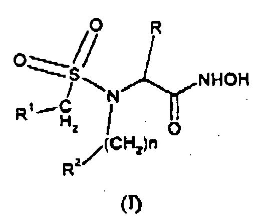 as inhibitors of CD23.

Mayer R J et al (Inflammation Research, 2002, 51, pp. 85 - 90) discusses CD23 shedding and its inhibition by dipeptide hydroxamic acids.

International patent application no. WO 01/62751 discloses a class of arylpiperazine and aryl piperidine derivatives as metalloproteinase (MMP) inhibitors, especially inhibitors of MMP-13.

International patent application no. WO 00/12478 discloses certain arylpiperazine derivatives as inhibitors of MMP.

### Summary of the Invention

We have now found a class of hydroxamate sulfonamides which are potent inhibitors of CD23 shedding. Therefore the compounds are particularly suitable for the treatment and/or prophylaxis of allergic diseases associated with IgE production.

Thus we provide a compound of formula (1): 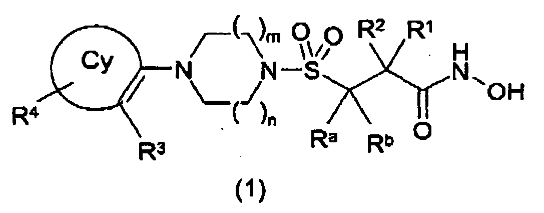 wherein:
Cy is an aryl or heteroaryl group;
m is zero or the integer 1, 2 or 3;
n is zero or the integer 1, 2 or 3; in which the sum of m and n is the integer 1, 2 or 3;
R¹ is a group selected from C₁₋₆alkyl, aryl, heteroaryl, heterocycloalkyl, C₃₋₆cycloalkyl, -C₁₋₆alkylaryl, -C₁₋₆alkylheteroaryl, -C₁₋₆ alkylheterocycloalkyl or -C₁₋₆alkylC₃₋₆cycloalkyl, in which each aryl or heteroaryl group, present as or as part of the group R¹, may optionally be substituted with 1, 2 or 3 substituents selected from the group R⁷, wherein each R⁷ may be the same or different, and is an atom or group selected from F, Cl, Br, C₁₋₆alkyl, C₁₋₆haloalkyl, C₁₋₆alkoxy, C₁₋₆haloalkoxy, -CN, -CO₂R^{7a}, -CON(R^{7a})₂ or -COR^{7a}; and in which each alkyl, heterocycloalkyl or cycloalkyl group, present as or as part of the group R¹, may optionally be substituted with 1, 2 or 3 substituents selected from the group R⁸, wherein each R⁸ may be the same or different, and is an atom or group selected from F, C₁₋₆alkyl, C₁₋₆haloalkyl, C₁₋₆alkoxy, C₁₋₆haloalkoxy, =O, =NOR¹⁰, -CO₂R^{8a}, -CON(R^{8a})₂ or -COR^{8a};
R^{7a}, which may be the same or different, is each a hydrogen atom, or a C₁₋₆alkyl or C₁₋₆haloalkyl group;
R^{8a}, which may be the same or different, is each a hydrogen atom, or a C₁₋₆alkyl or C₁₋₆haloalkyl group;
R¹⁰ is a hydrogen atom or a C₁₋₃alkyl group;
R² is a hydrogen atom or a C₁₋₃alkyl group;
or R¹ and R² together with the carbon atom to which they are attached form a C₃₋₆cycloalkyl or heterocycloalkyl group optionally substituted with 1, 2 or 3 substituents selected from the group R⁹, wherein each R⁹ may be the same or different, and is an atom or group selected from F, C₁₋₆alkyl, C₁₋₆haloalkyl, C₁₋₆alkoxy, C₁₋₆haloalkoxy, =O, =NOR¹⁰, -CO₂R^{8a}, -CON(R^{8a})₂ or -COR^{8a};
R³ is an atom or group selected from F, Cl, Br, C₁₋₃alkyl, C₁₋₃haloalkyl, C₁₋₃alkoxy or C₁₋₃haloalkoxy;
R⁴ is a hydrogen, F, Cl or Br atom or a C₁₋₃alkyl, C₁₋₃haloalkyl, C₁₋₃alkoxy, C₁₋₃haloalkoxy, -CN, -SO₂R⁵, -SO₂N(R⁶)₂, -CON(R⁶)₂, -N(R⁶)₂, -NHSO₂R⁵ or -NHCOR⁵ group;
R⁵ is a C₁₋₃alkyl group;
R⁶, which may be the same or different, is each a hydrogen atom or a C₁₋₃alkyl group; and
R^{a} and R^{b}, which may be the same or different, is each an atom or group selected from hydrogen or C₁₋₃alkyl, or R^{a} and R^{b} may be joined to form a C₃₋₆cycloalkyl or heterocycloalkyl group as defined for R¹ and R²;
and the salts, solvates, hydrates, tautomers, isomers or *N*-oxides thereof.

### Description of the Invention

It will be appreciated that certain compounds of formula (1) may exist as geometric isomers (E or Z isomers). The compounds may also have one or more chiral centres, and exist as enantiomers or diastereomers. The invention is to be understood to extend to all such geometric isomers, enantiomers, diastereomers and mixtures thereof, including racemates. Formula (1) and the formulae hereinafter are intended to represent all individual isomers and mixtures thereof, unless stated or shown otherwise. In addition, compounds of formula (1) may exist as tautomers, for example keto (CH₂C=O)-enol (CH=CHOH) tautomers.

It will also be appreciated that where desired the compounds of the invention may be administered in a pharmaceutically acceptable pro-drug form, for example as a protected hydroxamic acid derivative, e.g. as either *N*or *O*-substituted derivatives, such as *O*-benzoyl. It will be further appreciated that the pro-drugs may be converted *in vivo* to the active compounds of formula (1), and the invention is intended to extend to such pro-drugs.

In the compounds of the invention as represented by formula (1) and the more detailed description hereinafter certain of the general terms used in relation to substituents are to be understood to include the following atoms or groups unless specified otherwise.

Thus as used herein the term "C₁₋₆alkyl", whether present as a group or part of a group, refers to straight or branched C₁₋₆alkyl groups such as methyl, ethyl, *n*-propyl, isopropyl, *n*-butyl, isobutyl, *sec*-butyl, *tert*-butyl or neopentyl. The term "C₁₋₃alkyl" refers to a straight or branched C₁₋₃alkyl group selected from methyl, ethyl, *n*-propyl or isopropyl.

The term "C₃₋₆cycloalkyl group" refers to non-aromatic cyclic, saturated C₃₋₆ ring systems selected from cyclopropyl, cyclobutyl, cyclopentyl or cyclohexyl.

The term "heterocycloalkyl group" refers to a 3- to 10-membered saturated monocyclic or multicyclic hydrocarbon ring system containing one, two, or three L² linker atoms or groups. Particular examples of suitable L² atoms or groups include -O-, -S- and -N(R¹¹)-, where R¹¹ is a hydrogen atom or a C₁₋₆ alkyl group.

Particular examples of heterocycloalkyl groups include 3- to 7-membered monocyclic ring systems such as azetidinyl, tetrahydrofuranyl, tetrahydropyranyl, tetrahydrothiophenyl, tetrahydrothiopyranyl, pyrrolidinyl, oxazolidinyl, dioxolanyl, e.g. 1,3-dioxolanyl, imidazolidinyl, pyrazolidinyl, thiazolidinyl, piperidinyl, 1,4-dioxanyl, morpholinyl, 1,4-dithianyl, thiomorpholinyl, piperazinyl, *N*-C₁₋₆alkylpiperazinyl, *N*-C₁₋₆alkylpyrrolidinyl, *N-*C₁₋₆alkylpiperidinyl, *N*-C₁₋₆alkylmorpholinyl, homopiperazinyl or 7- to 10-membered multicyclic ring systems such as quinuclidinyl or 1,4-dioxaspiro[4.5]decane.

Typical heterocycloalkyl groups which may represent either R¹ and R² when joined together or R^{a} and R^{b} when joined together include 3- to 7-membered monocyclic ring systems, such as azetidinyl, tetrahydrofuranyl, tetrahydropyranyl, pyrrolidinyl and piperidinyl.

Heterocycloalkyl groups may be linked to the remainder of the compound of formula (1) by any available carbon atom or, when part of the group -C₁₋₆alkylheterocycloalkyl, by any carbon atom or heteroatom, e.g. nitrogen atom, as appropriate.

The term "halogen atom" is intended to include fluorine, chlorine, bromine or iodine atoms.

The term "C₁₋₆alkoxy" as used herein refers to straight or branched C₁₋₆alkoxy groups such as methoxy, ethoxy, *n-*propoxy, isopropoxy or *tert-*butoxy. Likewise the term "C₁₋₃alkoxy" as used herein refers to straight or branched C₁₋₃alkoxy groups such as methoxy, ethoxy, *n-*propoxy or isopropoxy.

The term "C₁₋₆haloalkoxy" as used herein includes any of those C₁₋₆alkoxy groups as defined herein substituted by one, two or three halogen atoms as described above. Similarly the term "C₁₋₃haloalkoxy" includes any of those C₁₋₃alkoxy groups as defined herein substituted by one, two or three halogen atoms as described above. Particular examples include -OCF₃, -OCCl₃, -OCHF₂, -OCHCl₂, -OCH₂F or -OCH₂Cl groups.

The term "aryl" refers to an aromatic carbocyclic radical having a single ring or two condensed rings. This term includes, for example, phenyl and naphthyl.

The term "heteroaryl" refers to a 5- to 10-membered aromatic monocyclic or multicyclic hydrocarbon ring system in which one, two or three atoms in the ring system is an element other than carbon, chosen from amongst nitrogen, oxygen or sulfur (or oxidised versions thereof, such as *N-*oxide). Monocyclic heteroaryl groups include, for example, five- or six-membered heteroaryl groups containing one, two or three heteroatoms selected from oxygen, sulfur or nitrogen atoms.

Particular examples of monocyclic ring heteroaryl groups of this type include pyrrolyl, furyl, thienyl, imidazolyl, *N*-C₁₋₆alkylimidazolyl, oxazolyl, isoxazolyl, thiazolyl, isothiazolyl, pyrazolyl, triazolyl, oxadiazolyl, thiadiazolyl, pyridyl, pyrimidinyl, pyridazinyl, pyrazinyl, tetrazolyl, triazinyl and pyridyl-*N-*oxide.

Particular examples of bicyclic ring heteroaryl groups of this type include benzofuryl, benzothienyl, indolyl, benzimidazolyl, benzothiazolyl, benzoxazolyl, benzisoxazolyl, quinazolinyl, quinoxalinyl, naphthyridinyl, pyrido[3,4-*b*]pyridyl, pyrido[3,2-*b*]pyridyl, pyrido[4,3-*b*]pyridyl, quinolinyl and isoquinolinyl.

The heteroaryl groups may be attached to the remainder of the compound of formula (1) by any available carbon atom.

The terms "-C₁₋₆alkylaryl", "-C₁₋₆alkylheteroaryl", "-C₁₋₆alkylheterocycloalkyl" and "C₁₋₆alkylC₃₋₆cycloalkyl" refer to a C₁₋₆alkyl group as defined herein in which a terminal hydrogen atom therein is replaced by an aryl, heteroaryl, heterocycloalkyl or C₃₋₆cycloalkyl group as described herein.

The presence of certain substituents in the compounds of formula (1) may enable salts of the compounds to be formed. Suitable salts include pharmaceutically acceptable salts, for example acid addition salts derived from inorganic or organic acids, and salts derived from inorganic and organic bases.

Acid addition salts include hydrochlorides, hydrobromides, hydroiodides, alkylsulphonates, e.g. methanesulphonates, ethanesulphonates, or isothionates, arylsulphonates, e.g. *p-*toluenesulphonates, besylates or napsylates, phosphates, sulphates, hydrogensulphates, acetates, trifluoroacetates, propionates, citrates, maleates, fumarates, malonates, succinates, lactates, oxalates, tartrates and benzoates.

Salts derived from inorganic or organic bases include alkali metal salts such as sodium or potassium salts, alkaline earth metal salts such as magnesium or calcium salts, and organic amine salts such as morpholine, piperidine, dimethylamine or diethylamine salts.

Particularly useful salts of compounds according to the invention include pharmaceutically acceptable salts, especially acid addition pharmaceutically acceptable salts.

One group of compounds of formula (1) has the formula (2): 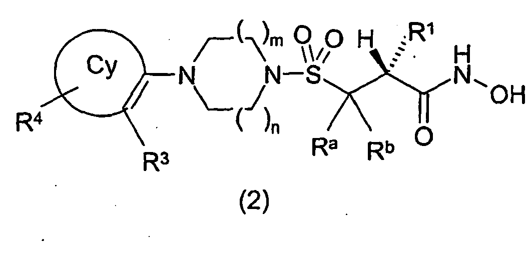 wherein m, n, Cy, R^{a}, R^{b}, R¹, R³ and R⁴ are as defined herein for compounds of formula (1);
and the salts, solvates, hydrates, tautomers, isomers or *N*-oxides thereof.

In one particular group of compounds of the invention Cy is a phenyl group or a monocyclic heteroaryl group, especially pyridyl, pyrimidinyl or pyrazinyl.

Cy is typically a phenyl group.

Another group of compounds of formula (1) has the formula (3): 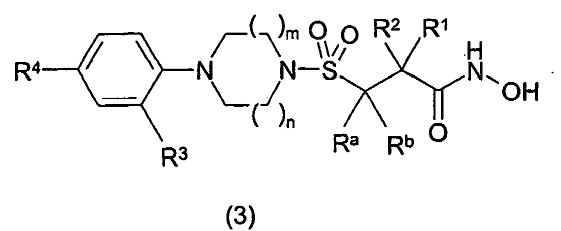 wherein m, n, R^{a}, R^{b}, R¹, R², R³ and R⁴ are as defined herein for compounds of formula (1);
and the salts, solvates, hydrates, tautomers, isomers or *N*-oxides thereof. One particular group of compounds of formula (3) has the formula (4): 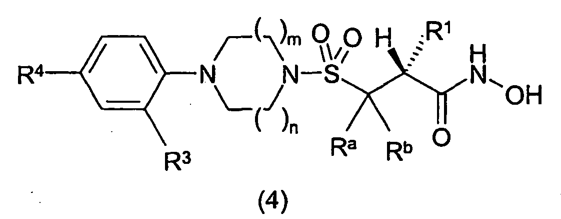 wherein m, n, R^{a}, R^{b}, R¹, R³ and R⁴ are as defined herein;
and the salts, solvates, hydrates, tautomers, isomers or *N*-oxides thereof.

In another particular aspect of the invention R^{a} and R^{b} is each a hydrogen atom.

In another particular aspect of the invention m is the integer 1 and n is the integer 1.

R² in one particular group of compounds of the invention is a hydrogen atom.

R¹ in one group of compounds of formula (1), (2), (3) or (4) is a group selected from C₁₋₆alkyl, phenyl, heteroaryl, heterocycloalkyl, C₃₋₆cycloalkyl, -(CH₂)₁₋₂phenyl, -(CH₂)₁₋₂heteroaryl, -(CH₂)₁₋₂heterocycloalkyl or -(CH₂)₁₋₂C₃₋₆cycloalkyl, in which each phenyl or heteroaryl group, present as or as part of the group R¹, may optionally be substituted with 1, 2 or 3 substituents selected from the group R⁷, as herein defined; and in which each alkyl, heterocycloalkyl or cycloalkyl group, present as or as part of the group R¹, may optionally be substituted with 1, 2 or 3 substituents selected from the group R⁸, as herein defined.

R¹ in a further group of compounds of formula (1), (2), (3) or (4) is a group selected from optionally substituted C₁₋₆alkyl, phenyl, heterocycloalkyl, C₃₋₆cycloalkyl or -(CH₂)₁₋₂phenyl.

Particular R¹ examples of this type include C₁₋₆alkyl, e.g. isopropyl, phenyl, pyridyl, pyrimidinyl, pyrrolyl, furyl, thienyl, imidazolyl, *N*-C₁₋₆alkyl-imidazolyl, oxazolyl, isoxazolyl, thiazolyl, isothiazolyl, tetrahydropyranyl, tetrahydrofuranyl, piperidinyl, pyrrolidinyl, 1,4-dioxaspiro[4.5]decanyl, cyclobutyl, cyclopentyl, cyclohexyl, -CH₂phenyl or -CH₂pyridyl.

R¹ in one particular group of compounds of formula (1), (2), (3) or (4) is an isopropyl, phenyl, 3,4-difluorophenyl, tetrahydropyranyl, cyclopentyl, -CH₂phenyl or -(CH₂)-3,4-difluorophenyl group, especially isopropyl, tetrahydropyranyl, phenyl or -CH₂phenyl. Typical examples of the group R¹ include isopropyl, -CH₂phenyl, cyclopentyl, phenyl and tetrahydropyranyl. Further typical examples include 3,4-difluorobenzyl, piperidin-4-yl, 1-methylpiperidin-4-yl, 1-ethylpiperidin-4-yl and 1-*tert*-butoxycarbonylpiperidin-4-yl.

In one group of compounds of the invention R⁷ is an atom or group selected from F, Cl, Br, C₁₋₆alkyl, C₁₋₆haloalkyl, C₁₋₆alkoxy, C₁₋₆haloalkoxy or -CN.

R⁷, in compounds of the invention, may be for example an atom or group selected from F, Cl, methyl, -CF₃, -CF₂H, methoxy, -OCF₃, -OCF₂H or -CN. Further examples of the group R⁷ include -CO₂H, -CO₂CH₃, -CO₂CH₂CH₃, -CO₂C(CH₃)₃, -CONH₂, -CON(H)CH₃, -CON(CH₃)₂ or -COCH₃. In one particular aspect of the invention R⁷ is a F atom.

In one group of compounds of the invention R⁸ is an atom or group selected from F, C₁₋₆alkyl, C₁₋₆haloalkyl, C₁₋₆alkoxy, C₁₋₆haloalkoxy, =O or =NOR¹⁰.

R⁸, in compounds of the invention, may be for example an atom or group selected from F, methyl, -CF₃, -CF₂H, methoxy, -OCF₃, -OCF₂H, =O, =NOH or =NOCH₃. Further examples of the group R⁸ include -CO₂H, -CO₂CH₃, -CO₂CH₂CH₃, -CO₂C(CH₃)₃, -CONH₂, -CON(H)CH₃, -CON(CH₃)₂ or -COCH₃ groups, especially -CO₂C(CH₃)₃. More particular examples of the group R⁸ include methyl, ethyl and -CO₂C(CH₃)₃.

Another group of compounds of the invention has the formula (1) or (3) wherein R¹ and R² together with the carbon atom to which they are attached form a C₃₋₆cycloalkyl group, particularly cyclobutyl, optionally substituted with R⁹ as defined herein.

In one group of compounds of the invention R⁹ is an atom or group selected from F, C₁₋₆alkyl, C₁₋₆haloalkyl, C₁₋₆alkoxy, C₁₋₆haloalkoxy, =O or =NOR¹⁰.

R⁹, in one group of compounds of the invention, is an atom or group selected from F, methyl, -CF₃, -CF₂H, methoxy, -OCF₃, -OCF₂H, =O =NOH or =NOCH₃.

Particular R³ examples include F, Cl, methyl, ethyl, isopropyl, -CF₃, -CF₂H, methoxy, ethoxy, -OCF₃ or -OCF₂H. R³, in one group of compounds of formula (1), (2), (3) or (4), is a F or Cl atom or a methyl, ethyl, -CF₃ or methoxy group, especially a F or Cl atom or a methyl, ethyl or methoxy group.

Particular R⁴ examples include hydrogen, F, Cl, methyl, ethyl, isopropyl, -CF₃, -CF₂H, methoxy, ethoxy, -OCF₃, -OCF₂H, -CN, -SO₂CH₃, -SO₂N(H)₂, -SO₂N(CH₃)₂, -SO₂N(H)CH₃, -CON(H)₂, -CON(CH₃)₂, -CON(H)CH₃, -N(H)₂, -N(CH₃)₂, -N(H)CH₃, -NHSO₂CH₃ or -NHCOCH₃. R⁴, in one group of compounds of formula (1), (2), (3) or (4), is a hydrogen, F or Cl atom or a methyl, -CF₃, methoxy, ethoxy, -OCF₃ or -OCF₂H group, especially a hydrogen, fluorine or chlorine atom or a methyl, ethoxy, -CF₃ or -OCF₃ group.

Certain compounds of the invention also have a surprisingly good selectivity for CD23 when compared with their ability to inhibit matrix metalloproteinases. Examples of such matrix metalloproteinases include MMP 9 or MMP 13. Such compounds are particularly useful for the treatment of diseases in which CD23 has a role, for example allergic and other diseases as described herein. Compounds of the invention which have this useful property include those of formula (1), (2), (3) or (4) wherein R³ is an atom or group selected from F, Cl, methyl ethyl or methoxy, especially F, Cl, methyl or methoxy. An especially preferred group of compounds is where R³ is a methyl group.

Compounds of this type include:
*N*-hydroxy-3-methyl-2-(4-*o*-tolylpiperazine-1-sulfonylmethyl)butyramide;
*N*-hydroxy-3-methyl-2-[4-(2-methyl-4-fluorophenyl)piperazine-1-sulfonylmethyl]butyramide;
*N*-hydroxy-3-methyl-2-[4-(2,4-dimethylphenyl)piperazine-1-sulfonylmethyl]-butyramide;
*N*-hydroxy-3-methyl-2-[4-(2-methyl-4-trifluoromethoxyphenyl)piperazine-1-sulfonylmethyl]butyramide;
2-benzyl-*N*-hydroxy-3-(4-*o*-tolylpiperazine-1-sulfonyl)propionamide;
2-benzyl-*N*-hydroxy-3-[4-(2-methyl-4-trifluoromethoxyphenyl)piperazine-1-sulfonyl]propionamide;
*N*-hydroxy-2-phenyl-3-(4-*o*-tolylpiperazine-1-sulfonyl)propionamide;
*N*-hydroxy-2(*R*)-(tetrahydropyran-4-yl)-3-(4-*o*-tolylpiperazine-1-sulfonyl)-propionamide;
*N*-hydroxy-3-methyl-2(*R*)-(4-*o*-tolylpiperazine-1-sulfonylmethyl)butyramide;
1-(4-*o*-tolylpiperazine-1-sulfonylmethyl)cyclobutanecarboxylic acid hydroxyamide;
and the salts, solvates, hydrates, tautomers, isomers or *N*-oxides thereof.

Further compounds of this type include:
*N*-hydroxy-3-methyl-2-[4-(2-methoxyphenyl)piperazine-1-sulfonylmethyl]-butyramide;
*N*-hydroxy-3-methyl-2-[4-(2-chlorophenyl)piperazine-1-sulfonylmethyl]-butyramide;
and the salts, solvates, hydrates, tautomers, isomers or *N*-oxides thereof.

Compounds of formula (1), (2), (3) or (4) are potent inhibitors of CD23 shedding. The ability of the compounds to act in this way may be simply determined by employing tests such as those described in the Examples hereinafter. The selectivity profile for certain compounds of the invention with respect to their inhibition of matrix metalloproteinases may be determined using the assay as described in Example D in the International Patent Application WO-A-98/05635.

Thus the compounds of the invention may be used in the treatment of conditions associated with increased levels of sCD23. The invention extends to such a use and in general to the use of the compounds of formula (1), (2), (3) or (4) for the manufacture of a medicament for treating such diseases and disorders.

Particular uses to which the compounds of the invention may be put include allergic diseases such as asthma, atopic dermatitis and other atopic diseases, allergic rhinitis, gastrointestinal allergies such as food allergies, eosinophilia, conjunctivitis, glomerular nephritis, graft-v-host disease, systemic anaphylaxis or hypersensitivity responses, urticaria, shock, drug allergies, insect sting allergies or parasite infections.

In a particular embodiment, the compounds of the present invention are useful for the treatment of the aforementioned exemplary disorders irrespective of their etiology, for example for the treatment of asthma, atopic dermatitis or allergic rhinitis.

Compounds of the invention may also be of use in other diseases where sCD23 is implicated including inflammatory diseases, such as rheumatoid arthritis or psoriasis, and neoplastic diseases, such as lymphoma or leukemia.

The compounds of formula (1), (2), (3) or (4) can be used alone or in combination with other compounds having related utilities to prevent and treat allergic disorders and diseases, including asthma and atopic dermatitis, as well as those pathologies as discussed herein.

For the prophylaxis or treatment of disease the compounds according to the invention may be administered as pharmaceutical compositions, and according to a further aspect of the invention we provide a pharmaceutical composition which comprises a compound of formula (1), (2), (3) or (4) together with one or more pharmaceutically acceptable carriers, excipients or diluents.

Alternative compositions of this invention comprise a compound of formula (1), (2), (3) or (4) or a salt thereof; an additional agent selected from an immunosuppressant or an anti-inflammatory agent; and any pharmaceutically acceptable carrier, adjuvant or vehicle.

Pharmaceutical compositions according to the invention may take a form suitable for oral, buccal, parenteral, nasal, topical, vaginal or rectal administration, or a form suitable for administration by inhalation or insufflation.

For oral administration, the pharmaceutical compositions may take the form of, for example, tablets, lozenges or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogenphosphate); lubricants (e.g. magnesium stearate, talc or silica); disintegrants (e.g. potato starch or sodium glycolate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents, emulsifying agents, non-aqueous vehicles and preservatives. The preparations may also contain buffer salts, and flavouring, colouring or sweetening agents, as appropriate.

Preparations for oral administration may be suitably formulated to give controlled release of the active compound.

For buccal administration the compositions may take the form of tablets or lozenges formulated in conventional manner.

The compounds for formula (1), (2), (3) or (4) may be formulated for parenteral administration by injection e.g. by bolus injection or infusion. Formulations for injection may be presented in unit dosage form, e.g. in glass ampoules or multi-dose containers, e.g. glass vials. The compositions for injection may take such forms as suspensions, solutions or emulsions in oily or aqueous vehicles, and may contain formulatory agents such as suspending, stabilising, preserving and/or dispersing agents. Alternatively, the active ingredient may be in powder form for constitution with a suitable vehicle, e.g. sterile pyrogen-free water, before use. For particle-mediated administration the compounds of formula (1), (2), (3) or (4) may be coated on particles such as microscopic gold particles.

In addition to the formulations described above, the compounds of formula (1), (2), (3) or (4) may also be formulated as a depot preparation. Such long-acting formulations may be administered by implantation or by intramuscular injection.

For nasal administration or administration by inhalation, the compounds for use according to the present invention are conveniently delivered in the form of an aerosol spray presentation for pressurised packs or a nebuliser, with the use of suitable propellant, e.g. dichlorodifluoromethane, trichlorofluoromethane, dichlorotetrafluoroethane, carbon dioxide or other suitable gas or mixture of gases.

For vaginal or rectal administration the compounds of formula (1), (2), (3) or (4) may be formulated as a suppository. These formulations may be prepared by mixing the active ingredient with a suitable non-irritating excipient which is a solid at room temperature but liquid at the body temperature. Such materials include, for example, cocoa butter and polyethylene glycols.

The compositions may, if desired, be presented in a pack or dispenser device which may contain one or more unit dosage forms containing the active ingredient. The pack or dispensing device may be accompanied by instructions for administration.

The quantity of a compound of the invention required for the prophylaxis or treatment of a particular condition will vary depending on the compound chosen, and the condition of the patient to be treated. In general, however, daily dosages may range from around 100 ng/kg to 100 mg/kg, e.g. around 0.01 mg/kg to 40 mg/kg body weight, for oral or buccal administration, from around 10 ng/kg to 50 mg/kg body weight for parenteral administration, and around 0.05 mg to around 1000 mg, e.g. around 0.5 mg to around 1000 mg, for nasal administration or administration by inhalation or insufflation.

The compounds according to the present invention may be used as pharmacological standards for use in the development of new biological tests and in the search for new pharmacological agents. The compounds according to the present invention may also be radiolabelled.

The compounds of the invention may be prepared by a number of processes as generally described below and more specifically in the Examples hereinafter. Many of the reactions described are well-known standard synthetic methods which may be applied to a variety of compounds and as such can be used not only to generate compounds of the invention but also, where necessary, the intermediates thereto.

In the following process description, the symbols m, n, Cy, R^{a}, R^{b}, R¹, R², R³ and R⁴, when used in the formulae depicted, are to be understood to represent those groups described above in relation to formula (1), (2), (3) or (4) unless otherwise indicated. In the reactions described below, it may be necessary to protect reactive functional groups, for example hydroxy, amino, thio or carboxy groups, where these are desired in the final product, to avoid their unwanted participation in the reactions. Conventional protecting groups may be used in accordance with standard practice [see, for example, Greene, T. W. in "Protective Groups in Organic Synthesis", John Wiley and Sons, (1999) and the examples herein]. In some instances, deprotection may be the final step in the synthesis of a compound of formula (1), (2), (3) or (4) and the processes according to the invention described hereinafter are to be understood to extend to such removal of protecting groups.

Thus, according to a further aspect of the invention, a compound of formula (1), or particular isomers thereof, may be prepared using the general method shown in Scheme A:

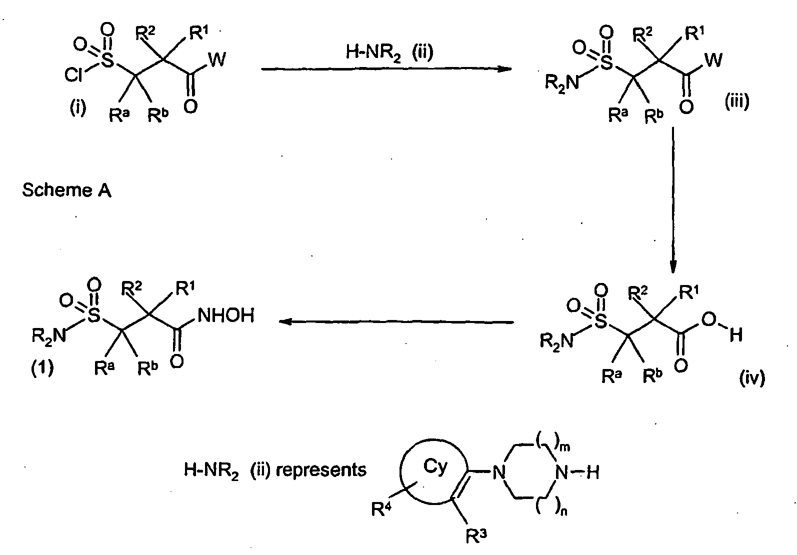

Thus, compounds of formula (iii), where W is, for example, an alkoxy group, such as methoxy, ethoxy or *tert-*butoxy, or a chiral auxiliary, for example 4-(*R*)-benzyloxazolidin-2-one may be prepared by methods well known in the literature, for example by reaction of a sulfonyl chloride (i) with an amine (ii) in the presence of an amine base, such as triethylamine, in a halogenated solvent, such as dichloromethane, at room temperature.

Compounds of general formula (i) are either known or may be made by one skilled in the art using conditions known in the literature, see for example WO-A-99/24399, or as described in the Examples hereinafter. Compounds of general formula (ii) are available commercially or they be made using methods known in the literature or by any method known to those skilled in the art.

Carboxylic acids of general formula (iv) may be prepared by deprotection of a suitably protected carboxylic acid of formula (iii). For example, where W is an alkoxy group, such as ethoxy, a base such as aqueous lithium hydroxide may be used. Alternatively, trifluoroacetic acid may be used when W is a *tert*-butyl group; or in the case of a chiral auxiliary, such as 4-(*R*)-benzyloxazolidin-2-one, lithium hydroxide/hydrogen peroxide may be used. Appropriate solvent and temperature conditions, such as those described in the Examples hereinafter, may be used.

Hydroxamic acids of general formula (1) may be prepared using conditions well known in the literature. For example, treatment of an acid of formula (iv) with oxalyl chloride in an inert solvent (such as dichloromethane) gives an intermediate acid chloride, which may or may not be isolated, but which in turn is reacted with hydroxylamine at a suitable temperature such as room temperature to give the desired hydroxamic acid (1). Alternatively, an acid of formula (iv) may be activated *in situ* using, for example, a diimide such as 1-(3-dimethylaminopropyl)-3-ethylcarbodiimide hydrochloride, advantageously in the presence of a catalyst such as a *N*-hydroxy compound, e.g. *N*-hydroxybenzotriazole, using suitable conditions, e.g. in *N*,*N*-dimethylformamide at -15°C, prior to the subsequent addition of a suitably protected hydroxylamine, such as *tert*-butyldimethylsilyl hydroxylamine, and warming to ambient temperature. The protecting group may be removed using appropriate conditions, such as water or tetrabutylammonium fluoride and acetic acid in tetrahydrofuran at 0°C, to yield the desired hydroxamic acid of formula (1).

Intermediates of formulae (i)-(iv) and any other intermediates required to obtain compounds of formula (1), (2), (3) or (4), when not available commercially, may be prepared by methods known to those skilled in the art following procedures set forth in references such as Rodd's Chemistry of Carbon Compounds, Volumes 1-15 and Supplementals (Elsevier Science Publishers, 1989), Fieser and Fieser's Reagents for Organic Synthesis, Volumes 1-19 (John Wiley and Sons, 1999), Comprehensive Heterocyclic Chemistry, Ed. Katritzky et al., Volumes 1-8, 1984, and Volumes 1-11, 1994 (Pergamon), Comprehensive Organic Functional Group Transformations, Ed. Katritzky et al., Volumes 1-7, 1995 (Pergamon), Comprehensive Organic Synthesis, Ed. Trost and Fleming, Volumes 1-9 (Pergamon, 1991), Encyclopedia of Reagents for Organic Synthesis, Ed. Paquette, Volumes 1-8 (John Wiley and Sons, 1995), Larock's Comprehensive Organic Transformations (VCH Publishers Inc., 1989), and March's Advanced Organic Chemistry (John Wiley and Sons, 1992).

Thus, for example, an amine of general formula (ii) may be prepared using methods known to those skilled in the art, including the general methods as shown in Scheme B:

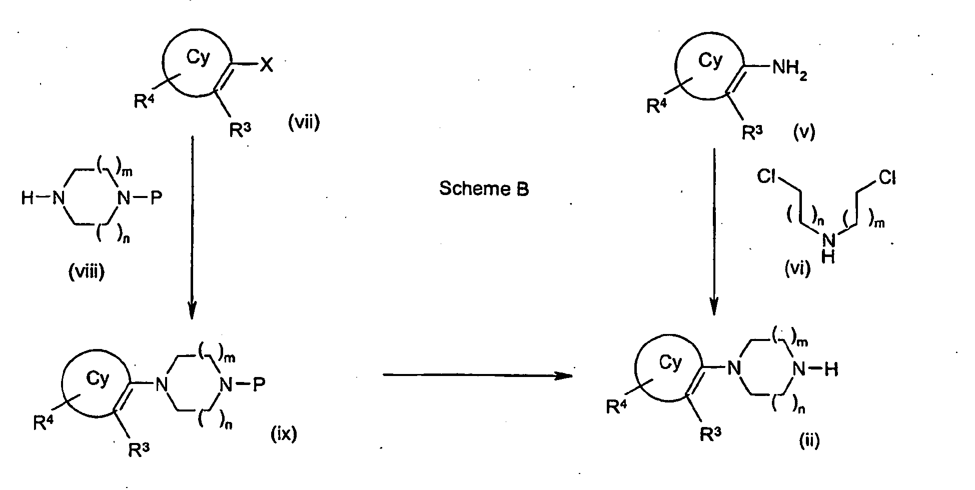

For example, when Cy is a phenyl group, an aniline of general formula (v) may be reacted with an amine of formula (vi) using known methodology, such as acid catalysis, in a suitable solvent, e.g. chlorobenzene, at elevated temperature, to give a compound of formula (ii).

Alternatively a suitably activated group of formula (vii) (where X is as defined below) may be reacted with an amine of formula (viii) (where P is a protecting group, e.g. *tert*-butoxycarbonyl) using standard methodology to give a compound of formula (ix). For example, when X is a halogen atom, e.g. bromine or iodine, or a suitable leaving group, e.g. trifluoromethylsulfonyloxy (OTf), or a boronic acid derivative appropriate conditions may involve the use of a palladium catalyst in a suitable solvent, e.g. tetrahydrofuran, at an elevated temperature. When X is a fluorine atom appropriate conditions may involve heating in an aprotic polar solvent, such as *N*-methylpyrrolidine, in the presence of a base, e.g. triethylamine. The compound of formula (ix) may be converted to a compound of formula (ii) using standard deprotection methods. It will be appreciated by those skilled in the art that different protecting groups P may be required at each stage of the synthesis in order to satisfy the reaction conditions and as such they may be interconverted using standard methods.

It will be appreciated that compounds of formula (1), (2), (3) or (4) or any preceding intermediates may be further derivatised by one or more standard synthetic methods employing substitution, oxidation, reduction or cleavage reactions. Particular substitution approaches include conventional alkylation, arylation, heteroarylation, acylation, thioacylation, halogenation, sulphonylation, nitration, formylation and coupling procedures. It will be appreciated that these methods may also be used to obtain or modify other compounds of formula (1), (2), (3) or (4) or any preceding intermediates where appropriate functional groups exist in these compounds.

Salts of compounds of formula (1), (2), (3) or (4) may be prepared by reaction of a compound of formula (1), (2), (3) or (4) with an appropriate base or acid in a suitable solvent or mixture of solvents, e.g. an organic solvent such as an ether, e.g. diethyl ether, or an alcohol, e.g. ethanol, or an aqueous solvent, using conventional procedures. Salts of compounds of formula (1), (2), (3) or (4) may be exchanged for other salts by use of conventional ionexchange chromatography procedures.

Where it is desired to obtain a particular enantiomer of a compound of formula (1), (2), (3) or (4) this may be produced from a corresponding mixture of enantiomers using any suitable conventional procedure for resolving enantiomers.

Thus, for example, diastereomeric derivatives, e.g. salts, may be produced by reaction of a mixture of enantiomers of formula (1), (2), (3) or (4), e.g. a racemate, and an appropriate chiral compound, e.g. a chiral base. The diastereomers may then be separated by any convenient means, for example by crystallisation, and the desired enantiomer recovered, e.g. by treatment with an acid in the instance where the diastereomer is a salt.

In another resolution process a racemate of formula (1), (2), (3) or (4) may be separated using chiral High Performance Liquid Chromatography. Alternatively, if desired, a particular enantiomer may be obtained by using an appropriate chiral intermediate in one of the processes described above.

Chromatography, recrystallisation and other conventional separation procedures may also be used with intermediates or final products where it is desired to obtain a particular geometric isomer of the invention.

The following Examples illustrate the invention. All temperatures are in °C. Where experimental detail is not given for the preparation of a reagent it is either commercially available, or it is known in the literature, for which the CAS number is quoted. The compounds are named with the aid of Beilstein Autonom supplied by MDL Information Systems GmbH, Theodor-Heuss-Allee 108, D-60486 Frankfurt, Germany.

¹H NMR spectra were obtained at 300 MHz or 400 MHz unless otherwise indicated.

The following LCMS conditions were used to obtained the retention times (RT) as described herein:

### LCMS conditions:

HP1100 (Diode Array) linked to a Finnigan LC-Q Mass Spectrometer, ESI mode with Pos/Neg ionisation.

| | |
|---|---|
| Column: | Luna C18(2) 100 x 4.6 mm, 5 µm particle size Analytical column |
| Column Temp: | 35°C |
| Mobile Phase: | A: Water + 0.08% formic acid |
| | B: Acetonitrile + 0.1 % formic acid |
| Flow rate: | 3 ml/min |

| Gradient: | Time (min): | % Composition B: |
|---|---|---|
| | 0 | 5 |
| | 4.4 | 95 |
| | 5.30 | 95 |
| | 5.32 | 5 |
| | 6.5 | 5 |
| Run time: | 6.5 min | |
| Typical Injection Volume: | 5 µl | |
| Detector Wavelength: DAD | | 205-330 nm |

### Preparative LC conditions:

### Gilson 215 liquid handler setup.

| | |
|---|---|
| Column: | Luna C18(2) 250 x 21.2 mm, 5 µm particle size PREP column |
| Column Temp: | Ambient |
| Mobile Phase: | A: Water + 0.08% formic acid |
| | B: Acetonitrile + 0.1 % formic acid |
| Gradient: | Variable - depends on retention of sample in LCMS screen |
| Run Time: | 20 min |
| Flow rate: | 20 ml/min |
| Typical Injection Volume: | 750 µl of 25 mg/ml solution |
| Detector Wavelength: | 210 and 254 nm |

Abbreviations used:

| | |
|---|---|
| DCM - dichloromethane | THF - tetrahydrofuran |
| MeOH - methanol | DMF - *N*,*N*-dimethylformamide |
| TFA - trifluoroacetic acid | MTBE - *tert*-butyl methyl ether |
| nBuLi - *n-*butyllithium | pTSA - *p*-toluenesulfonic acid |
| Hunig's base - *N*,*N*-diisopropylethylamine | |
| CDCl₃ - deuterated chloroform | d₆DMSO - deuterated dimethylsulfoxide |
| Methanol-d₄ - deuterated methanol | |

### Intermediate 1

### 3-Methyl-2-methylenebutyric acid

Isopropyl malonic acid (30 g) was dissolved in 1,4-dioxane (200 ml) and piperidine (30 ml) was added, followed by aqueous formaldehyde (30 ml). The solution was stirred overnight and the resulting thick white suspension was heated to 100°C for 2 h, then cooled and evaporated. The mixture was diluted with water (300 ml) and washed with ether (200 ml), then acidified with citric acid to pH 4 and extracted with DCM (2 x 200 ml). The solvent was washed with water (300 ml) and brine (300 ml), dried and evaporated to give the title compound as a colourless solid (25 g). MS 114 (M).

### Intermediate 2

### 2-Bromomethyl-3-methylbutyric acid

3-Methyl-2-methylenebutyric acid (25 g) was dissolved in 48% hydrobromic acid in acetic acid (100 ml) and the solution stirred overnight at room temperature, then added to water (300 ml) and extracted with diethyl ether. The mixture was extracted with diethyl ether and the solvent washed with water and brine, dried and evaporated to give the title compound as a pale amber solid (33 g). MS 195 (M).

### Intermediate 3

### 2-Bromomethyl-3-methylbutyric acid tert-butyl ester

2-Bromomethyl-3-methylbutyric acid (33 g) was placed in a Parr pressure reactor, cooled to -78°C and isobutylene (200 ml) and DCM (200 ml) were added, followed by concentrated sulphuric acid (1 ml). The vessel was sealed and the mixture stirred at room temperature for 18 h, then pressure carefully released and the solution added to saturated sodium bicarbonate solution. The mixture was extracted with diethyl ether, the solvent washed with water and brine and evaporated *in vacuo* to give the title compound as a colourless liquid (33 g). MS 251 (M).

### Intermediate 4

### 2-Acetylsulfanylmethyl-3-methylbutyric acid tert-butyl ester

Potassium thioacetate (20 g) was added to a solution of 2-bromomethyl-3-methylbutyric acid *tert*-butyl ester (33 g) in DMF (200 ml) and the brown mixture stirred for 18 h, then added to water (1 litre), and the mixture extracted with diethyl ether: The solvent was washed with water, saturated sodium bicarbonate solution and brine, dried and evaporated to give the title compound as an amber oil (29 g). MS 246 (M).

### Intermediate 5

### 2-Chlorosulfonylmethyl-3-methylbutyric acid tert-butyl ester

Chlorine was passed through a solution of 2-acetylsulfanylmethyl-3-methylbutyric acid *tert*-butyl ester (29 g) in DCM (100 ml) and water (100 ml) at 0°C for 1 h, giving a pale green solution. The phases were separated and the organic layer washed with water, sodium bicarbonate solution and brine, dried and evaporated to give the product as a colourless liquid which crystallised on refrigeration (27 g). MS 270 (M).

### Intermediate 6

### (Tetrahydropyran-4-ylidene)acetic acid methyl ester

Carbomethoxy triphenylphosphonium bromide (45 g) was added to a solution of tetrahydropyran-4-one (10 g) in THF. Sodium hydride (4.2 g) was added carefully in small portions. The suspension was stirred at reflux for 18 h, then cooled, filtered and evaporated. The residue was filtered through silica, eluting with diethyl ether/hexane 1:1 to give the title compound as a colourless oil (13 g). MS 156 (M).

### Intermediate 7

### (Tetrahydropyran-4-yl)acetic acid methyl ester

(Tetrahydropyran-4-ylidene)acetic acid methyl ester (13 g) was hydrogenated at atmospheric pressure in methanol for 24 h, the solution filtered and evaporated to give the title compound as a colourless liquid (13 g). MS 158 (M).

### Intermediate 8

### (Tetrahydropyran-4-yl)acetic acid

Sodium hydroxide (16 g) in water (400, ml) was added to a solution of (tetrahydropyran-4-yl)acetic acid methyl ester (13 g) in methanol. The mixture was stirred overnight at room temperature, then evaporated *in vacuo*. The solution was washed with diethyl ether, acidified with concentrated hydrochloric acid to pH 2 and extracted with ethyl acetate, the solvent washed with brine, dried and evaporated to give the title compound as a colourless solid (10.2 g). MS 144 (M).

### Intermediate 9

### 4(R)-Benzyl-3-[2-(tetrahydropyran-4-yl)acetyl]oxazolidin-2-one

Oxalyl chloride (5 ml) and DMF (1 drop) were added to a solution of (tetrahydropyran-4-yl)acetic acid (10 g) in DCM. The mixture was stirred for 3 h, then evaporated *in vacuo* and thoroughly azeotroped with toluene. The residue was dissolved in THF and added dropwise to a solution of (*R*)-benzyloxazolidinone (12.1 g) and nBuLi (2.5M in hexanes, 30 ml) in THF (200 ml) at -78°C. The solution was stirred for 2 h, then quenched with saturated aqueous ammonium chloride and evaporated *in vacuo*. The mixture was extracted with ethyl acetate, solvent washed with water and brine, dried and evaporated to give the title compound as a colourless solid (14 g). MS 304 (M + H).

### Intermediate 10

### 4(R)-Benzyl-3-[3-hydroxy-2(S)-(tetrahydropyran-4-yl)propionyl]-oxazolidin-2-one

Titanium tetrachloride (14 ml, 1M in DCM) was added to a solution of 4(*R*)-benzyl-3-[2-(tetrahydropyran-4-yl)acetyl]oxazolidin-2-one (4 g) in DCM (100 ml) at 0°C, followed by Hunig's base (2.5 ml). The mixture was stirred for 30 min, then trioxane (1.2 g) and titanium tetrachloride (14 ml) were added. The dark purple suspension was stirred for 4 h, then quenched with saturated ammonium chloride solution, the organic layer washed with water and brine, dried and evaporated. The residue was columned (ether) to give the title compound as a white solid (1.6 g). MS 334 (M + 1).

### Intermediate 11

### 4(R)-Benzyl-3-[3-iodo-2(R)-(tetrahydropyran-4-yl)propionyl]oxazolidin-2-one

4(*R*)-Benzyl-3-[3-hydroxy-2(*S*)-(tetrahydropyran-4-yl)propionyl]-oxazolidin-2-one (1.6 g) was dissolved in toluene and triphenylphosphine (1.4 g), iodine (1.3 g) and imidazole (350 mg) were added. The mixture was stirred at reflux for 1 h, then cooled, washed with water and the solution evaporated. The residue was columned (2:1 diethyl ether:hexane) to give the title compound as a white solid (1.8 g). MS 444 (M + 1).

### Intermediate 12

### 4(R)-Benzyl-3-[3-acetylsulfanyl-2(R)-(tetrahydropyran-4-yl)propionyl]-oxazolidin-2-one

4(*R*)-Benzyl-3-[3-iodo-2(*R*)-(tetrahydropyran-4-yl)propionyl]oxazolidin-2-one (1.8 g) was dissolved in DMF (20 ml) and potassium thioacetate (600 mg) was added. The suspension was stirred for 4 h, then added to water and extracted with ethyl acetate. The solvent was washed with water, bicarbonate and brine, dried and evaporated to give the title compound as a pale orange gum (1.5 g). MS 392 (M + H).

### Intermediate 13

### 3-(4(R)-Benzyl-2-oxooxazolidin-3-yl)-3-oxo-2(R)-(tetrahydropyran-4-yl)propane-1-sulfonyl chloride

Chlorine was passed through a solution of 4(*R*)-benzyl-3-[3-acetylsulfanyl-2(*R*)-(tetrahydropyran-4-yl)propionyl]oxazolidin-2-one (1.5 g) in DCM (100 ml) and water (100 ml) for 30 min. The solution was stirred for 30 min, purged with nitrogen and the phases separated. The organic layer was washed with water and brine, dried and evaporated to give the title compound as a colourless solid (1.3 g). MS 416 (M + 1).

### Intermediate 14

### 2-Benzylacrylic acid

Prepared from benzyl malonic acid (25 g), using the method as described for 3-methyl-2-methylenebutyric acid, to give the title compound as a white solid (18 g). MS 162 (M + 1).

### Intermediate 15

### 2-Bromomethyl-3-phenylpropionic acid

Prepared from 2-benzylacrylic acid (18 g), using the method as described for 2-bromomethyl-3-methylbutyric acid, to give the title compound as a white solid (23 g). MS 243 (M).

### Intermediate 16

### 2-Bromomethyl-3-phenylpropionic acid tert-butyl ester

Prepared using the method as described for 2-bromomethyl-3-methylbutyric acid *tert*-butyl ester from 2-bromomethyl-3-phenylpropionic acid (23 g) to give the title compound as a brown oil (28 g). MS 299.

### Intermediate 17

### 2-Acetylsulfanylmethyl-3-phenylpropionic acid tert-butyl ester

Prepared using the method as described for 2-acetylsulfanylmethyl-3-methylbutyric acid *tert*-butyl ester from 2-bromomethyl-3-phenylpropionic acid *tert*-butyl ester (28 g) to give the title compound as a yellow oil (18.5 g). MS 294 (M).

### Intermediate 18

### 2-(Chlorosulfonylmethyl)-3-phenylpropionic acid tert-butyl ester

Prepared using the method as described for 2-chlorosulfonylmethyl-3-methylbutyric acid *tert*-butyl ester from 2-acetylsulfanylmethyl-3-phenylpropionic acid *tert*-butyl ester (18.5 g) as a colourless solid (19 g). MS 319 (M + H).

### Intermediate 19

### 2-Cyclopentylacrylic acid

Prepared using the method as described for 3-methyl-2-methylenebutyric acid from cyclopentylmalonic acid (5 g) to give the title compound as a yellow oil (4.1 g). 1H NMR (δH, CDCl₃) 11.50 (1H, s), 6.30 (1 H, s), 5.80 (1 H, s), 2.95 (1 H, q), 1.95-2.00 (2H, m), 1.65-1.80 (4H, m), 1.35-1.50 (2H, m).

### Intermediate 20

### 3-Bromo-2-cyclopentylpropionic acid

Prepared using the method as described for 2-bromomethyl-3-methylbutyric acid from 2-cyclopentylacrylic acid (4.1 g) to give the title compound as a white solid (4.34 g). 1H NMR (5H, CDCl₃) 10.50 (1H, s), 3.45-3.65 (2H, m), 2.55-2.75 (1H, m), 1.90-2.15 (1H, m), 1.70-1.90 (2H, m), 1.45-1.70 (4H, m), 1.15-1.45 (2H, m). MS 221 (M).

### Intermediate 21

### 3-Acetylsulfanyl-2-cyclopentylpropionic acid

Potassium thioacetate (2.24 g) was added to a solution of 3-bromo-2-cyclopentylpropionic acid (4.34 g) in DMF (20 ml) and the mixture stirred for 24 h. The brown solution was added to water, extracted with diethyl ether and the solvent washed with water and brine, dried and evaporated *in vacuo* to give the title compound as a brown solid (3.8 g). 1 H NMR (δH, CDCl₃) 3.30 (1H, dd), 2.96-3.00 (1 H, m), 2.50 (1 H, dd), 2.38 (3H, s), 2.05 (1 H, q), 1.85-1.95 (1 H, m), 1.45-1.70 (4H, m), 1.25-1.40 (2H, m).

### Intermediate 22

### 3-Acetylsulfanyl-2-cyclopentylpropionic acid tert-butyl ester

3-Acetylsulfanyl-2-cyclopentylpropionic acid (3.8 g) was dissolved in a mixture of isobutylene (30 ml) and DCM (30 ml), concentrated sulphuric acid (1 ml) was added and the mixture stirred in a Parr pressure reaction vessel for 18 h. The pressure was released cautiously and the solution added to saturated sodium bicarbonate solution, the phases separated and the organic layer washed with water and brine, dried and evaporated to give the title compound as a brown oil (4.1 g). 1 H NMR (δH, CDCl₃) 3.35 (1H, dd), 3.10-3.25 (1H, m), 2.45 (1H, dd), 2.40 (3H, s), 2.05 (1H, q), 1.85-1.95 (1H, m), 1.40-1.65 (4H, m), 1.30 (9H, s), 1.25-1.40 (2H, m).

### Intermediate 23

### 3-Chlorosulfonyl-2-cyclopentylpropionic acid tert-butyl ester

Prepared using the method as described for 2-chlorosulfonylmethyl-3-methylbutyric acid *tert*-butyl ester from 3-acetylsulfanyl-2-cyclopentyl-propionic acid *tert*-butyl ester (1.7 g) to give the title compound as an amber oil (1.6 g). 1 H NMR (δH, CDCl₃) 4.25 (1 H, dd), 3.70 (1 H, dd), 2.90 (1 H, dt), 2.05 (1 H, m), 1.85-1.95 (1 H, m), 1.40-1.65 (4H, m), 1.30 (9H, s), 1.25-1.40 (2H, m).

### Intermediate 24

### 1-(Chlorosulfonylmethyl)cyclobutanecarboxylic acid ethyl ester

*n-*Butyllithium (49.8 ml of 1.6M solution in hexanes) was added to a solution of diisopropylamine (11.2 ml) in THF (90 ml) at -78°C and the solution stirred for 30 min. A solution of ethyl cyclobutanecarboxylate (10 ml) was added dropwise and the mixture stirred for 30 min, then treated with diiodomethane (6.4 ml). The mixture was stirred for 3 h and allowed to warm to room temperature, quenched with water (50 ml) and evaporated. The residual mixture was partitioned between water and ethyl acetate, the organic layer washed with water and brine, dried and evaporated. The residue was dissolved in DMF (50 ml) and potassium thioacetate (8.3 g) was added. The brown solution was stirred overnight at room temperature, then added to water and extracted with ethyl acetate. The solvent was washed with water and brine, dried and evaporated to a brown oil. The residue was dissolved in DCM (100 ml), water (100 ml) was added and chlorine bubbled through the mixture at 0°C. The organic layer was washed with water and brine, dried and evaporated to give the title compound as a brown oil (9.8 g). TLC R_{f} 0.45 (2:1 heptane-ethyl acetate).

### Intermediate 25

### 4(R)-Benzyl-3-(3-methylbutyryl)oxazolidin-2-one

*n-*Butyllithium (2.5M in hexanes, 65 ml) was added to a solution of (*R*)-benzyloxazolidinone (28.9 g) in THF (200 ml) at -78°C and the mixture was stirred for 30 min, then 3-methylbutanoyl chloride (22 ml) was added and the solution stirred for 2 h. The reaction mixture was quenched with saturated ammonium chloride, evaporated *in vacuo* and the residue extracted with DCM (2 x 200 ml). The solvent was washed with water, bicarbonate solution and brine, dried and evaporated to give the title compound as a colourless solid (41.5 g). MS 261 (M).

### Intermediate 26

### 4(R)-Benzyl-3-(2(S)-hydroxymethyl-3-methylbutyryl)oxazolidin-2-one

Titanium tetrachloride (18 ml) was added to a solution of 4(*R*)-benzyl-3-(3-methylbutyryl)oxazolidin-2-one (41.5 g) in DCM at 0°C. Hunig's base (28 ml) was added and the purple solution stirred for 30 min, then a solution of trioxane (11.2 g) in DCM was added dropwise, followed by titanium tetrachloride. The mixture was stirred vigorously for 2 h at 0°C, giving an amber solution, which was quenched with saturated aqueous ammonium chloride. The phases were separated and the organic layer washed with water, bicarbonate solution and brine, dried and evaporated to a white solid (45 g). MS 291 (M).

### Intermediate 27

### 4(R)-Benzyl-3-(2(R)-iodomethyl-3-methylbutyryl)oxazolidin-2-one

Iodine (42 g), triphenylphosphine (47 g) and imidazole (12 g) were added to a solution of 4(*R*)-benzyl-3-(2(*S*)-hydroxymethyl-3-methylbutyryl)-oxazolidin-2-one (45 g) in toluene (500 ml) and the mixture was boiled under reflux for 1 h. The resulting suspension was cooled, filtered and the filtrate washed with water and brine. The solid residue was dissolved in DCM and filtered through silica (200 g) eluting with ether/hexane to give the title compound as a pale yellow oil (57 g). MS 401 (M).

### Intermediate 28

### 4(R)-Benzyl)-3-(2(R)-acetylthiomethyl-3-methylbutyryl)oxazolidin-2-one

Potassium thioacetate (19 g) was added to a solution of 4(*R*)-benzyl-3-(2(*R*)-iodomethyl-3-methylbutyryl)oxazolidin-2-one (56 g) in DMF (300 ml) and the mixture was stirred at room temperature for 3 h, then added to water (2 1) and extracted with ether (2 x 500 ml). The solvent was washed with water, bicarbonate solution and brine, dried and evaporated to give the title compound as a pale amber oil (49 g). MS 349 (M).

### Intermediate 29

### 4(R)-Benzyl-3-(2(R)-chlorosulfonylmethyl-3-methylbutyryl)oxazolidin-2-one

Chlorine was bubbled through a solution of 4(*R*)-benzyl-3-(2(*R*)-acetylthiomethyl-3-methylbutyryl)oxazolidin-2-one (49 g) in DCM (200 ml) and water (200 ml) until the solution became yellow. The mixture was stirred vigorously for 1 h, then purged with nitrogen, the phases were separated and the organic layer washed with water and brine, dried and evaporated to give the title compound as a colourless gum (42 g). MS 373 (M). 1H NMR (δH, CDCl₃) 7.20-7.40 (5H, m), 4.65-4.80 (2H, m), 4.45 (1 H, dd), 4.20 (2H, d), 3.70 (1H, dd), 3.45 (1 H, dd), 2.65 (1 H, dd), 2.10 (1 H, m), 1.15 (3H, d), 0.03 (3H, d).

### Intermediate 30

### 1-(2-Methyl-4-ethoxyphenyl)piperazine

2-Methyl-4-ethoxyaniline (2.3 g) and bis-chloroethylamine hydrochloride (3 g) were heated in chlorobenzene (200 ml) with pTSA (2.6 g) for 24 h. The mixture was cooled and evaporated *in vacuo* and the residue columned on silica eluting with 12% MeOH/DCM containing 1% ammonium hydroxide to give the title compound as a pink solid (0.45 g). MS 221 (M + H).

### Intermediate 31

### 1-(2-Methyl-4-trifluoromethoxyphenyl)piperazine

Prepared from 2-methyl-4-trifluoromethoxyaniline using the method as described for 1-(2-methyl-4-ethoxyphenyl)piperazine to give the title compound as a white solid (0.55 g). MS 261 (M + H).

### Intermediate 32

### 1-(2-Fluoro-4-trifluoromethylphenyl)piperazine

1-*tert* Butoxycarbonylpiperazine (1.9 g) was added to a solution of 3,4-difluorobenzotrifluoride (1.9 g) in NMP (20 ml) and triethylamine (1.5 ml) and the mixture was heated at 120°C for 72 h, then cooled, added to water (80 ml) and extracted with DCM (100 ml). The solvent was washed with water (100 ml) dried and evaporated. The residue was dissolved in DCM (50 ml) and treated with TFA (10 ml), the solution stirred for 2 h, then evaporated *in vacuo* and the residue dissolved in water (100 ml). The aqueous solution was washed with ether (30 ml), basified with 1 M sodium hydroxide and extracted with DCM (100 ml), solvent washed with water (50 ml), dried and evaporated to give the title compound as a colourless solid (300 mg). MS 249 (M + H).

### Intermediate 33

### 3-Bromo-2-phenylpropionic acid

Prepared from phenylmalonic acid [CAS number 492-38-6] (4 g) following the procedure as described for 2-bromomethyl-3-methylbutyric acid to yield an amber oil (5.2 g). MS 229 (M).

### Intermediate 34

### 3-Bromo-2-phenylpropionic acid tert-butyl ester

Prepared using the method as described for 2-bromomethyl-3-methylbutyric acid *tert*-butyl ester from 3-bromo-2-phenylpropionic acid (5 g) as a colourless oil (4.5 g). MS 285 (M).

### Intermediate 35

### 3-Acetylsulfanyl-2-phenylpropionic acid tert-butyl ester

Prepared using the method as described for 2-acetylsulfanylmethyl-3-methylbutyric acid *tert*-butyl ester from 3-bromo-2-phenylpropionic acid-*tert-*butyl ester (4 g) as a yellow liquid (3.3 g). MS 280 (M).

### Intermediate 36

### 3-Chlorosulfonyl-2-phenylpropionic acid tert-butyl ester

Prepared using the method as described for 2-chlorosulfonylmethyl-3-methylbutyric acid *tert*-butyl ester from 3-acetylsulfanyl-2-phenylpropionic acid *tert*-butyl ester (3 g) as a beige solid (2.1 g). TLC R_{f} 0.47 (diethyl ether).

### Intermediate 37

### 4(R)-Benzyl-3-[3-(3,4-difluorophenyl)propionyl]oxazolidin-2-one

3,4-Difluorophenylhydrocinnamic acid (10 g, 53 mmol) was dissolved in DCM (100 ml) and stirred with oxalyl chloride (10 ml) and DMF (1 drop) for 3 h at room temperature. The solution was evaporated *in vacuo* and azeotroped with heptane (2 x 200 ml). The residue was dissolved in THF (20 ml) and added dropwise to a solution of (*R*)-benzyloxazolidinone (9 g) and nBuLi (1.6M in hexanes, 35 ml) in THF (100 ml) at -78°C. The mixture was stirred for 2 h, quenched with saturated ammonium chloride solution (100 ml), evaporated *in vacuo* and the solid product collected by filtration to give the title compound as a colourless solid (16 g). MS 346 (M + 1). TLC R_{f} 0.65 (Et₂O).

### Intermediate 38

### 4(R)-Benzyl-3-[3-(3,4-difluorophenyl)-2(S)-hydroxymethylpropionyl]-oxazolidin-2-one

Intermediate 37 (6.9 g) was dissolved in dry DCM (150 ml) at 0°C and titanium tetrachloride (2.2 ml) was added, followed by Hunig's base (3.5 ml). The mixture was stirred for 2 h, and then quenched with saturated ammonium chloride (100 ml). The phases were separated and the organic layer washed with saturated sodium bicarbonate solution (2 x 100 ml) and brine, dried and evaporated and the residue purified by column chromatography on silica (3:1 Et₂O/hexanes) to give the title compound as a colourless solid (4.3 g). MS 376 (M + 1). TLC R_{f} 0.45 (Et₂O).

### Intermediate 39

### 4(R)-Benzyl-3-[3-(3,4-difluorophenyl)-2(R)-iodomethylpropionyl]-oxazolidin-2-one

Intermediate 38 (4.3 g) was suspended in toluene (100 ml) and triphenylphosphine (3 g), iodine (2.9 g) and imidazole (1 g) were added. The mixture was heated at reflux for 1 h, cooled and washed with water (100 ml), saturated sodium bicarbonate solution (100 ml) and brine, dried and evaporated. The residue was filtered through a silica plug eluting with ether-hexane (1:1) to give the title compound as a colourless gum (3.7 g). TLC R_{f} 0.35 (1:1 ether-hexane).

### Intermediate 40

### Thioacetic acid [3-(4(R)-benzyl-2-oxooxazolidin-3-yl)-2(R)-(3,4-difluorobenzyl)-3-oxopropyl] ester

Intermediate 39 (3.7 g) was dissolved in DMF (50 ml) and potassium thioacetate (0.95 g) was added. The mixture was stirred at room temperature for 3 h, added to water and extracted with Et₂O (100 ml). The solution was washed with water (2 x 50 ml), and the residue was purified by column chromatography on silica eluting with 2:1 Et₂O-hexane to give the title compound as a pale yellow oil (3.05 g). TLC R_{f} 0.45 (2:1 Et₂O-hexane).

### Intermediate 41

### 3-(4(R)-Benzyl-2-oxooxazolidin-3-yl)-2(R)-(3,4-difluorobenzyl)-3-oxopropane-1-sulfonyl chloride

Intermediate 40 (3.05 g) was dissolved in DCM (50 ml) and water (40 ml) and chlorine was bubbled through the solution at 0°C for 10 min. The pale yellow mixture was stirred for 30 min, then the phases separated and the organic layer washed with water and brine (50 ml), dried and evaporated to give the title compound as a colourless solid (3.10 g). TLC R_{f} 0.54 (Et₂O). 1H NMR (δH, CDCl₃) 6.90-7.30 (8H, m), 5.00 (1H, m), 4.60 (1H, m), 4.40 (1H, dd), 4.10-4.20 (2H, m), 3.60 (1H, dd), 3.40 (1H, dd), 3.20 (1H, dd), 2.70-2.80 (2H, m).

### Intermediate 42

### 1-tert-Butoxycarbonylpiperidin-4-ylmalonic acid

Titanium tetrachloride (22 ml) was added dropwise to a solution of 1-*tert*-butoxycarbonylpiperidin-4-one (20 g) and diethyl malonate (16 ml) in THF (200 ml) at 0°C. Pyridine (52 ml) was added dropwise and the mixture was stirred overnight. Water (500 ml) and EtOAc (500 ml) were added, the organic layer washed with brine (300 ml) and 1 M HCl (300 ml), dried and evaporated. The residue was dissolved in ethanol (200 ml) and hydrogenated at atmospheric pressure over 10% Pd/C (2 g) overnight. The mixture was filtered and aqueous sodium hydroxide (2M, 200 ml) was added. The solution was boiled under reflux for 6 h, cooled, evaporated and the residue partitioned between 1 M HCl (400 ml) and EtOAc (400 ml). The solvent was dried and evaporated and the residue triturated with Et₂O to give the title compound as a white crystalline solid (9 g). TLC R_{f} 0.27 (EtOAc/1% AcOH).

### Intermediate 43

### 4-(1-Carboxyvinyl)piperidine-1-carboxylic acid tert-butyl ester

Intermediate 42 (9 g) was dissolved in 1,4-dioxane (60 ml) and formaldehyde solution (37% aq., 10 ml) and piperidine (10 ml) were added. The mixture was stirred overnight, and then heated at reflux for 1 h. The solution was evaporated *in vacuo* and partitioned between 1 M HCl (100 ml) and Et₂O (100 ml). The solvent was washed with water and brine (50 ml), dried and evaporated to give the title compound as a colourless crystalline solid (5.6 g). TLC R_{f} 0.42 (Et₂O).

### Intermediate 44

### 4-[1-(4(R)-Benzyl-2-oxooxazolidine-3-carbonyl)vinyl]piperidine-1-carboxylic acid tert-butyl ester

Intermediate 43 (4.0 g) was dissolved in DCM (50 ml) and pyridine (3 ml) and treated with oxalyl chloride (3 ml) and DMF (1 drop). The solution was stirred for 3 h, then evaporated *in vacuo* and azeotroped to dryness with heptane. The product was dissolved in THF (20 ml) and added dropwise to a solution of 4(*R*)-benzyloxazolidin-2-one (2.7 g) and nBuLi (2.5M in hexanes, 6.5 ml) in THF (60 ml) at -78°C. The mixture was stirred for 4 h, and then quenched with ammonium chloride solution (200 ml), extracted with EtOAc (200 ml) and the solvent washed with water and brine (50 ml), dried and evaporated. The residue was purified by column chromatography on silica eluting with 3:1 Et₂O-hexane to give the title compound as a colourless solid (3.3 g). TLC R_{f} 0.35 (3:1 Et₂O-hexanes). 1H NMR (δH, CDCl₃) 7.20-7.40 (5H, m), 5.40 (2H, m), 4.75 (1H, m). 4.10-4.35 (4H, m), 3.30 (1H, dd), 2.85 (1H, dd), 2.70 (2H, dt), 2.55 (1H, dt), 1.85 (2H, dt), 1.60 (9H, s), 1.45-1.60 (2H, m).

### Intermediate 45

### 4-[1(R)-Acetylsulfanylmethyl-2-(4(R)-benzyl-2-oxooxazolidin-3-yl)-2-oxoethyl]piperidine-1-carboxylic acid tert-butyl ester

Intermediate 44 (3.3 g) was stirred in thioacetic acid (10 ml) for 18 h at room temperature. The mixture was diluted with Et₂O (100 ml) and washed with 1 M NaOH (2 x 50 ml), water and brine, dried and evaporated. Analysis showed the crude product to be a 9 to 1 mixture of diastereomers. The residue was purified by column chromatography on silica eluting with 1:1 Et₂O/hexane to give the title compound as a white solid (2.6 g). TLC R_{f} 0.27 (1:1 Et₂O/hexane). 1H NMR (δH, CDCl₃) 7.20-7.40 (5H, m), 4.70 (1H, m), 4.004.20 (5H, m), 3.25-3.40 (2H, m), 3.10 (1H, dd), 2.75 (1H, dd), 2.55-2.70 (2H, m), 2.35 (3H, s), 1.90 (1H, m), 1.20-1.70 (4H, m), 1.45 (9H, s).

### Intermediate 46

### 4-[2-(4(R)-Benzyl-2-oxooxazolidin-3-yl)-1(R)-chlorosulfonylmethyl-2-oxoethyl]piperidine-1-carboxylic acid tert-butyl ester

Chlorine was bubbled through a solution of Intermediate 45 (1.6 g) and sodium acetate (5 g) in DCM (50 ml) and water (20 ml) at 0°C for 10 min, until a faint yellow colour persisted in the organic layer. The mixture was stirred for a further 30 min, then the phases were separated and the organic layer washed with water and brine, dried and evaporated to give the title compound as a colourless solid (1.6 g). TLC R_{f} 0.53 (Et₂O). 1H NMR (δH, CDCl₃) 7.20-7.40 (5H, m), 4.80 (1H, m), 4.70 (1H, m), 4.45 (1H, dd), 4.15-4.30 (4H, m), 3.80 (1H, dd), 3.50 (1H, dd), 2.65 (1H, dd), 2.55-2.70 (2H, m), 1.90 (1H, m), 1.70 (2H, m), 1.45 (9H, s), 1.35-1.55 (2H, m).

### Method A

### Example 1

### N-Hydroxy-3-methyl-2-(4-o-tolylpiperazine-1-sulfonylmethyl)butyramide

1-*ortho*-Tolylpiperazine (106 mg) was added to a solution of 2-chlorosulfonylmethyl-3-methylbutyric acid *tert*-butyl ester (135 mg) and triethylamine (0.25 ml) in DCM (10 ml) and the solution was stirred for 2 h at room temperature. TFA (2 ml) was added and the mixture was stirred for 3 h, then evaporated *in vacuo* and azeotroped to dryness with toluene. The residue was dissolved in DCM (20 ml) and oxalyl chloride (2 ml) and DMF (1 drop) were added. The solution was stirred for 2 h, then evaporated to dryness and azeotroped to dryness with toluene. The residue was dissolved in THF (10 ml) and aqueous hydroxylamine (1 ml) was added. After stirring for 1 h, the mixture was diluted with water (10 ml) and evaporated to half volume *in vacuo.* The product was collected by filtration, washed with water (5 ml) and dried to give the title compound as a beige solid (55 mg). TLC R_{f} 0.42 (diethyl ether). MS 370 (M + 1). 1H NMR (δH, d₆DMSO) 10.50 (1H, s), 8.80 (1H, s), 7.10-7.40 (4H, m), 3.70 (1H, dd), 3.40 (4H, m), 3.10 (5H, m), 2.50 (1H, m), 2.30 (3H, s), 1.90 (1H, m), 0.98 (3H, d), 0.95 (3H, d).

### Example 2

### N-Hydroxy-3-methyl-2-(4-o-fluorophenylpiperazine-1-sulfonylmethyl)-butyramide

Prepared using Method A from 2-chlorosulfonylmethyl-3-methylbutyric acid *tert*-butyl ester (100 mg) and 1-(2-fluorophenyl)piperazine (100 mg) as a white solid (33.8 mg). MS 374 (M + H). 1H NMR (δH, d₆DMSO) 10.60 (1H, s), 8.90 (1H, s), 7.00-7.30 (3H, m), 3.55 (1H, dd), 3.30 (4H, m), 3.10 (5H, m), 2.40 (1H, dt), 1.85 (1H, dq), 0.95 (6H, appears as doublet).

### Example 3

### N-Hydroxy-3-methyl-2-[4-(2,4-difluorophenyl)piperazine-1-sulfonylmethyl]butyramide

Prepared using Method A from 2-chlorosulfonylmethyl-3-methylbutyric acid *tert*-butyl ester (100 mg) and 1-(2,4-difluorophenyl)piperazine (100 mg) as a colourless solid (26.3 mg). MS 392 (M + H). 1H NMR (δH, d₆DMSO) 10.50 (1H, s), 8.80 (1H, s), 7.20 (1H, m), 6.90-7.10 (2H, m), 3.50 (1H, dd), 3.25 (4H, m), 2.90-3.05 (5H, m), 2.30 (1H, dt), 1.70 (1H, dq), 0.80 (6H, appears as doublet).

### Example 4

### N-Hydroxy-3-methyl-2-[4-(2-methyl-4-fluorophenyl)-piperazine-1-sulfonylmethyl]butyramide

Prepared using Method A from 2-chlorosulfonylmethyl-3-methylbutyric acid *tert*-butyl ester (100 mg) and 1-(2-methyl-4-fluorophenyl)piperazine as a white solid (25 mg). MS 388 (M + H). 1H NMR (δH, CDCl₃) 8.50 (2H, br s), 6.80-7.10 (3H, m), 3.50 (1H, dd), 3.40 (4H, m), 3.10 (1H, dd), 2.90 (4H, m), 2.30 (3H, s), 2.10 (1 H, m), 1.70 (1 H, m), 1.00 (6H, appears as triplet).

### Example 5

### N-Hydroxy-3-methyl-2-[4-(2,4-dimethylphenyl)piperazine-1-sulfonylmethyl]butyramide

Prepared using Method A from 2-chlorosulfonylmethyl-3-methylbutyric acid *tert*-butyl ester (135 mg) and 1-(2,4-dimethylphenyl)piperazine (95 mg) as a beige solid. MS 384 (M + H). TLC R_{f} 0.47 (8% MeOH, DCM). 1H NMR (δH, d₆DMSO) 10.60 (1H, s), 8.80 (1H, s), 7.20 (1H, d), 7.10 (1H, s), 7.00 (1H, d), 3.60 (1H, dd), 3.30 (4H, m), 3.10 (5H, m), 2.40 (3H, s), 2.30 (1H, m), 2.10 (3H, s), 1.80 (1H, m), 0.90 (6H, appears as triplet).

### Example 6

### N-Hydroxy-3-methyl-2-[4-(2,3-dimethylphenyl)piperazine-1-sulfonylmethyl]butyramide

Prepared using Method A from 2-chlorosulfonylmethyl-3-methylbutyric acid *tert*-butyl ester (135 mg) and 1-(2,3-dimethylphenyl)piperazine (95 mg) as a beige solid (60 mg). MS 384 (M + H). 1H NMR (δH, d₆DMSO) 10.50 (1H, s), 8.60 (1H, s), 6.90-7.20 (3H, m), 3.60 (1H, dd), 3.30 (4H, m), 3.10 (5H, m), 2.25 (3H, s), 2.20 (1H, m), 2.10 (3H, s), 1.80 (1H, m), 0.90 (6H, appears as triplet).

### Example 7

### N-Hydroxy-3-methyl-2-[4-(2-methoxyphenyl)piperazine-1-sulfonylmethyl]butyramide

Prepared using Method A from 2-chlorosulfonylmethyl-3-methylbutyric acid *tert*-butyl ester (135 mg) and 1-(2-methoxyphenyl)piperazine (96 mg) as a beige solid (55 mg). MS 386 (M + H). 1H NMR (δH, d₆DMSO) 10.50 (1H, s), 8.90 (1H, s), 6.80-7.20 (4H, m), 3.80 (3H, s), 3.60 (1H, dd), 3.40 (4H, m), 2.90-3.10 (5H, m), 2.30 (1H, m), 1.80 (1H, m), 0.90 (6H, appears as triplet).

### Example 8

### N-Hydroxy-3-methyl-2-[4-(2-chlorophenyl)piperazine-1-sulfonylmethyl]-butyramide

Prepared using Method A from 2-chlorosulfonylmethyl-3-methylbutyric acid *tert*-butyl ester (135 mg) and 1-(2-chlorophenyl)piperazine (117 mg) as a white solid (42 mg). MS 390 (M + H). 1 H NMR (δH, d₆DMSO) 10.60 (1 H, s), 8.90 (1H, s), 7.00-7.30 (4H, m), 3.70 (1H, dd), 3.30 (4H, m), 2.90-3.10 (5H, m), 2.30 (1 H, m), 1.80 (1 H, m), 0.90 (6H, appears as doublet).

### Example 9

### N-Hydroxy-3-methyl-2-[4-(2-ethylphenyl)piperazine-1-sulfonylmethyl]-butyramide

Prepared using Method A from 2-chlorosulfonylmethyl-3-methylbutyric acid *tert*-butyl ester (135 mg) and 1-(2-ethylphenyl)piperazine (95 mg) as a beige solid (45 mg). MS 384 (M + H). 1 H NMR (δH, d₆DMSO) 10.60 (1 H, s), 8.90 (1H, s), 6.80-7.20 (4H, m), 3.70 (1 H, dd), 3.30 (4H, m), 2.90-3.10 (5H, m), 2.40 (1 H, m), 2.30 (2H, q), 1.80 (1 H, m), 1.00 (3H, t), 0.90 (6H, appears as doublet).

### Example 10

### N-Hydroxy-3-methyl-2-[4-(2-fluoro-4-trifluoromethylphenyl)piperazine-1-sulfonylmethyl]butyramide

Prepared using Method A from 2-chlorosulfonylmethyl-3-methylbutyric acid *tert*-butyl ester (160 mg) and 1-(2-fluoro-4-trifluoromethylphenyl)-piperazine (200 mg) to give the title compound as a white solid (65 mg). MS 442 (M + H). ¹H NMR (δH, d₆DMSO) 10.80 (1H, s), 9.10 (1H, s), 7.80 (1H, d), 7.70 (1H, d), 7.40 (1H, t), 3.70 (1H, dd), 3.60 (4H, m), 3.40 (4H, m), 3.30 (1H, dd), 2.50 (1H, dt), 2.00 (1H, dq), 1.00 (6H, appears as doublet).

### Example 11

### N-Hydroxy-3-methyl-2-[4-(2-methyl-4-ethoxyphenyl)piperazine-1-sulfonylmethyl]butyramide

Prepared using Method A from 1-(2-methyl-4-ethoxyphenyl)piperazine (150 mg) and 2-chlorosulfonylmethyl-3-methylbutyric acid *tert*-butyl ester (130 mg) as a beige solid (7 mg). MS 414 (M + H). ¹H NMR (δH, d₆DMSO) 10.50 (1H, s), 8.80 (1H, s), 7.20 (1H, d), 7.00 (1H, s), 6.80 (1H, d), 3.90 (2H, q), 3.70 (1H, dd), 3.60 (4H, m), 3.30 (1H, dd), 3.20 (4H, m), 2.50 (1H, dt), 2.00 (1H, dq), 1.30 (3H, q), 1.00 (6H, appears as doublet).

### Example 12

### N-Hydroxy-3-methyl-2-[4-(2-methyl-4-trifluoromethoxyphenyl)piperazine-1-sulfonylmethyl]butyramide

Prepared using Method A from 1-(2-methyl-4-trifluoromethoxyphenyl)-piperazine (140 mg) and 2-chlorosulfonylmethyl-3-methylbutyric acid *tert*-butyl ester (135 mg) as a white solid (25 mg). MS 454 (M + 1). ¹H NMR (δH, d₆DMSO) 10.50 (1H, s), 8.80 (1H, s), 7.40 (1H, d), 7.20 (1H, s), 6.90 (1H, d), 3.70 (1H, dd), 3.60 (4H, m), 3.40 (4H, m), 3.30 (1H, dd), 2.50 (1H, dt), 1.30 (3H, q), 1.00 (6H, appears as doublet).

### Example 13

### 2-Benzyl-N-hydroxy-3-[4-(2-methoxyphenyl)piperazine-1-sulfonyl]-propionamide

Prepared using Method A from 2-(chlorosulfonylmethyl)-3-phenylpropionic acid *tert*-butyl ester (200 mg) and 1-(2-methoxyphenyl)piperazine (143 mg) as a colourless solid (61 mg). MS 434 (M + H). 1H NMR (δH, CDCl₃) 8.40 (2H, br s), 7.20-7.40 (4H, m), 7.15 (1H, m), 7.00 (1H, m), 6.80-6.90 (3H, m), 3.85 (3H, s), 3.60 (1H, dd), 3.30 (4H, m), 3.10 (4H, m), 2.80-3.00 (3H, m).

### Example 14

### 2-Benzyl-3-[4-(2-fluorophenyl)piperazine-1-sulfonyl]-N-hydroxypropionamide

Prepared using Method A from 2-(chlorosulfonylmethyl)-3-phenylpropionic acid *tert*-butyl ester (200 mg) and 1-(2-fluorophenyl)piperazine (140 mg) as a white solid (84 mg). MS 422 (M + H). 1H NMR (δH, d₆DMSO) 10.70 (1 H, br s), 8.90 (1 H, br s), 7.00-7.40 (9H, m), 3.50 (1 H, dd), 3.25 (4H, m), 3.10 (4H, m), 2.75-3.10 (4H, m).

### Example 15

### 2-Benzyl-3-[4-(2,4-difluorophenyl)piperazine-1-sulfonyl]-N-hydroxypropionamide

Prepared using Method A from 2-(chlorosulfonylmethyl)-3-phenylpropionic acid *tert*-butyl ester (200 mg) and 1-(2,4-difluorophenyl)piperazine as a white solid (10 mg). MS 440 (M + H). 1H NMR (δH, d₆DMSO) 10.80 (1H, s), 8.90 (1H, s), 7.25-7.60 (6H, m), 7.00-7.20 (2H, m), 3.60 (1H, dd), 3.20 (4H, m), 2.70-3.10 (8H, m).

### Example 16

### 2-Benzyl-N-hydroxy-3-(4-o-tolylpiperazine-1-sulfonyl)propionamide

Prepared using Method A from 2-(chlorosulfonylmethyl)-3-phenylpropionic acid *tert*-butyl ester (320 mg) and 1-(2-methylphenyl)piperazine (200 mg) as a white solid (130 mg). MS 418 (M + H). 1H NMR (δH, d₆DMSO) 10.80 (1H, s), 8.90 (1H, s), 6.90-7.20 (9H, m), 3.60 (1H, dd), 3.20 (4H, m), 2.70-3.10 (8H, m), 2.30 (3H, s).

### Example 17

### 2-Benzyl-N-hydroxy-3-[4-(2-methyl-4-trifluoromethoxyphenyl)piperazine-1-sulfonyl]propionamide

Prepared using Method A from 2-(chlorosulfonylmethyl)-3-phenylpropionic acid *tert*-butyl ester (130 mg) and Intermediate 30 (130 mg) as a beige solid (85 mg). MS 502 (M + H). 1H NMR (δH, d₆DMSO) 10.90 (1H, s), 8.70 (1H, s), 6.90-7.60 (8H, m), 3.50 (1H, dd), 3.20 (4H, m), 2.80-3.20 (8H, m), 2.20 (3H, s).

### Example 18

### 2-Benzyl-3-[4-(4-ethoxy-2-methylphenyl)piperazine-1-sulfonyl]-N-hydroxypropionamide

Prepared using Method A from 2-(chlorosulfonylmethyl)-3-phenylpropionic acid *tert*-butyl ester (150 mg) and 1-(2-methyl-4-ethoxyphenyl)-piperazine (130 mg) as a beige solid (90 mg). MS 462 (M + H). 1H NMR (δH, d₆DMSO) 10.80 (1H, s), 8.70 (1H, s), 6.90-7.40 (8H, m), 3.70 (2H, q), 3.50 (1H, dd), 3.20 (4H, m), 2.80-3.20 (8H, m), 2.20 (3H, s), 1.40 (3H, t).

### Example 19

### 2-Cyclopentyl-3-[4-(2,4-difluorophenyl)piperazine-1-sulfonyl]-N-hydroxypropionamide

Prepared using Method A from 3-chlorosulfonyl-2-cyclopentylpropionic acid *tert*-butyl ester (162 mg) and 1-(2,4-difluorophenyl)piperazine (100 mg) as a beige solid (25 mg). MS 418 (M + H). 1H NMR (δH, d₆DMSO) 7.05-7.20 (1H, m), 6.80-7.00 (2H, m), 3.65 (1H, dd), 3.40 (4H, m), 3.10 (5H, m), 2.40 (1H, dt), 1.90-2.10 (2H, m). 1.60-1.90 (5H, m), 1.10-1.40 (2H, m).

### Example 20

### N-Hydroxy-2-phenyl-3-(4-o-tolylpiperazine-1-sulfonyl)propionamide

Prepared using Method A from 3-chlorosulfonyl-2-phenylpropionic acid *tert*-butyl ester (160 mg) and 1-(2-methylphenyl)piperazine (230 mg) as a beige solid (22 mg). MS 404 (M + H). 1H NMR (δH, d₆DMSO) 10.90 (1H, s), 9.00 (1H, s), 4.00 (1H, dd), 3.85 (1 H, dd), 3.30 (1H, dd), 3.20 (4H, m), 2.80 (4H, m), 2.20 (3H, s).

### Method B

### Example 21

### N-Hydroxy-2(R)-(tetrahydropyran-4-yl)-3-(4-o-tolylpiperazine-1-sulfonyl)-propionamide

1-*ortho*-Tolylpiperazine was added to a solution of 3-(4(*R*)-benzyl-2-oxooxazolidin-3-yl)-3-oxo-2(*R*)-(tetrahydropyran-4-yl)propane-1-sulfonyl chloride (210 mg) and triethylamine (0.16 ml) in DCM (10 ml) at 0°C and the solution was stirred for 3 h, then washed with water (10 ml), citric acid solution (10 ml) and brine (10 ml), dried and evaporated. The residue was dissolved in THF (10 ml) and hydrogen peroxide (8M aq., 0.14 ml) was added. The mixture was cooled to 0°C and a solution of lithium hydroxide (22 mg) in water (5 ml) was added dropwise over 20 min. The mixture was stirred for 3 h, allowed to warm to room temperature and quenched with aqueous sodium sulphite (5% aq., 10 ml). The mixture was evaporated, washed with ether (10 ml) and the aqueous layer acidified with citric acid and extracted with DCM (20 ml). The solvent was washed with water and brine, dried and evaporated and the residue dissolved in dry DCM (10 ml). To the solution was added oxalyl chloride (0.3 ml) and DMF (1 drop) and the mixture stirred for 2 h, then evaporated to dryness and the residue dissolved in THF (3 ml). Aqueous hydroxylamine (0.3 ml) was added, the solution stirred for 30 min, then evaporated and the residue triturated with water (5 ml) to give the title compound as a white solid (105 mg). TLC R_{f} 0.50 (5% MeOH/DCM). MS 412 (M + 1). 1H NMR (δH, d₆DMSO) 10.70 (1H, s), 7.20-7.30 (2H, m), 7.00-7.10 (2H, m), 3.96 (2H, m), 3.55 (1H, dd), 3.20-3.40 (7H, m), 3.00 (4H, m), 2.50 (1H, dt), 2.35 (3H, s), 1.70-1.90 (2H, m), 1.20-1.50 (3H, m).

### Example 22

### N-Hydroxy-3-methyl-2(R)-(4-o-tolylpiperazine-1-sulfonylmethyl)-butyramide

Prepared using Method B from 4(*R*)-benzyl-3-(2(*R*)-chlorosulfonylmethyl-3-methylbutyryl)oxazolidin-2-one (650 mg) and 1-(2-methylphenyl)-piperazine (320 mg) as a white solid (230 mg). MS 370 (M + H). 1 H NMR (δH, d₆DMSO) 10.70 (1 H, s), 9.00 (1 H, s), 7.15-7.25 (2H, m), 6.95-7.10 (2H, m), 3.50 (1 H, dd), 3.30 (4H, m), 3.10 (1H, dd), 2.90 (4H, m), 2.50 (1 H, dt), 2.30 (3H, s), 1.90 (1 H, m), 0.95 (6H, appears as doublet).

### Method C

### Example 23

### 1-(4-o-Tolylpiperazine-1-sulfonylmethyl)cyclobutanecarboxylic acid hydroxyamide

1-(2-Methylphenyl)piperazine (150 mg) was added to a solution of 1-(chlorosulfonylmethyl)cyclobutane carboxylic acid ethyl ester (170 mg) in DCM and triethylamine (0.3 ml) was then added. The mixture was stirred for 3 h, then washed with citric acid solution, bicarbonate solution and brine, dried and evaporated. The residue was dissolved in THF (10 ml) and aqueous lithium hydroxide (0.2 g in 5 ml water) was added, the solution stirred overnight, diluted with water (10 ml) and evaporated. The mixture was washed with ether (5 ml), then acidified and extracted with DCM (20 ml). The solvent was washed with water (20 ml) and brine (20 ml), dried and evaporated and the residue dissolved in DCM (10 ml). Oxalyl chloride (0.3 g) was added, followed by DMF (1 drop). The mixture was stirred for 1 h, then evaporated and the residue azeotroped to dryness with toluene. The solid was dissolved in THF (10 ml) and hydroxylamine solution added (1 ml), the solution stirred overnight, then diluted with DCM (30 ml) and washed with water (30 ml). The solvent was dried and evaporated and the solid triturated with diethyl ether (10 ml) to give the title compound as a white solid (70 mg). MS 368 (M + H). 1 H NMR (δH, CDCl₃) 8.80 (2H, br s), 7.20 (2H, m), 7.00 (2H, m), 3.60 (2H, s), 3.40 (4H, m), 3.00 (4H, m), 2.30-2.50 (4H, m), 2.20 (3H, s), 2.10 (2H, m).

### Example 24

### 1-[4-(2-Fluorophenyl)piperazine-1-sulfonylmethyl]cyclobutane carboxylic acid hydroxyamide

Prepared using Method C from 1-(2-fluorophenyl)piperazine (100 mg) and 1-(chlorosulfonylmethyl)cyclobutane carboxylic acid ethyl ester (100 mg) as a white solid (10 mg) after purification by preparative HPLC. MS 372 (M + 1). 1 H NMR (δH, d₆DMSO) 10.50 (1H, s), 8.80 (1H, s), 6.90-7.20 (4H, m), 3.65 (2H, s), 3.30 (4H, m), 3.10 (4H, m), 2.20-2.50 (4H, m), 1.70-2.00 (2H, m).

### Example 25

### 1-[4-(2,4-Difluorophenyl)piperazine-1-sulfonylmethyl]cyclobutane carboxylic acid hydroxyamide

Prepared using Method C from 1-(2,4-difluorophenyl)piperazine (120 mg) and 1-(chlorosulfonylmethyl)cyclobutane carboxylic acid ethyl ester (100 mg) as a white solid (11.7 mg). MS 390 (M + H). 1H NMR (δH, d₆DMSO) 10.80 (1H, s), 9.00 (1H, s), 7.10-7.40 (3H, m), 3.86 (2H, s), 3.45 (4H, m), 3.20 (4H, m), 2.20-2.50 (4H, m), 1.95-2.20 (2H, m).

### Example 26

### 3-(3,4-Difluorophenyl)-2(R)-[4-(2,4-difluorophenyl)piperazine-1-sulfonylmethyl]-N-hydroxypropionamide

Prepared by Method B from Intermediate 41 (230 mg) and 4-(2,4-diflurophenyl)piperazine (115761-79-0) (100 mg) to give the title compound as a colourless solid (85 mg). 1H NMR (δH, d₆DMSO) 10.70 (1H, s), 8.90 (1H, s), 7.00-7.40 (6H, m), 3.60 (1H, dd), 3.30 (4H, m), 3.10 (5H, m), 2.90 (3H, m). MS 476 (M + 1).

### Example 27

### 4-[1-Hydroxycarbamoyl-2(R)-(4-ortho-tolylpiperazine-1-sulfonyl)ethyl]-piperidine-1-carboxylic acid tert-butyl ester

Prepared by Method B from Intermediate 46 (260 mg) and 4-(*ortho-*tolyl)piperazine hydrochloride (55974-34-0) (120 mg) as a colourless solid (60 mg). MS 511 (M + 1). HPLC retention time 3.59 min. TLC R_{f} 0.60 (7% MeOH/DCM).

### Example 28

### N-Hydroxy-2(R)-(piperidin-4-yl)-3-(4-ortho-tolylpiperazine-1-sulfonyl)-propionamide, trifluoroacetate salt

Example 27 (50 mg) was dissolved in DCM (10 ml) and TFA (1 ml) was added. The solution was stirred for 2 h, then evaporated *in vacuo* and azeotroped to dryness with DCM/heptane. The product was crystallised from methanol (0.5 ml) and Et₂O (5 ml) to give the title compound as a beige crystalline solid (35 mg). MS 411 (M + 1) (free base). 1H NMR (δH, CD₃OD) 7.10-7.40 (4H, m), 3.75 (1H, dd), 3.60 (4H, m), 3.30 (1H, dd), 3.10 (4H, m), 2.75 (1 H, m), 2.50 (3H, s), 2.00-2.25 (2H, m), 1.60-1.95 (2H, m).

### Example 29

### 2(R)-(1-Ethylpiperidin-4-yl)-N-hydroxy-3-(4-ortho-tolylpiperazine-1-sulfonyl)propionamide

Acetaldehyde (200 mg) was added to a mixture of Example 28 (20 mg) and activated 4A molecular sieves (100 mg) in DCM (2 ml) and the suspension was stirred for 2 h, then sodium triacetoxyborohydride (60 mg) was added. The mixture was stirred overnight, then filtered and washed with sodium bicarbonate (2 ml) and brine (2 ml), the solvent dried and evaporated and the product purified by preparative HPLC to give the title compound as a colourless solid (1.5 mg). MS 439 (M + 1). HPLC retention time 1.88 min.

### Example 30

### N-Hydroxy-2(R)-(1-methylpiperidin-4-yl)-3-(4-ortho-tolylpiperazine-1-sulfonyl)propionamide

Prepared from Example 28 (47 mg), formaldehyde (37% aq., 0.12 ml) and sodium triacetoxyborohydride (0.12 g) as a beige solid (28 mg). 1H NMR (δH, CD₃OD) 6.90-7.30 (4H, m), 3.50 (1H, dd). 3.40 (4H, m), 3.14 (1H, dd), 2.90 (4H, m), 2.85 (1H, m), 2.50 (1H, m), 2.30 (3H, s), 2.25 (3H, s), 2.20-2.30 (2H, m), 2.00-2.10 (2H, m), 1.85 (1H, m), 1.30-1.70 (3H, m). MS 425 (M + 1).

The ability of the compounds of the invention to inhibit the shedding of CD23 may be determined using the following assays:

Abbreviations used:

| | |
|---|---|
| DTT - Dithiothreitol | CO₂ - Carbon Dioxide |
| FCS - Foetal Calf Serum | IL-4 - Interleukin-4 |
| ELISA - Enzyme-Linked ImmunoSorbent Assay | |

### Plasma Membrane CD23 Shedding Assay

Plasma membranes were isolated from RPMI8866 cells by initially resuspending the cells in 20 mM Hepes buffer (+ NaCl 150 nM, MgCl₂ 1.5 mM at pH 7.5 containing DTT 1 mM) and homogenising in a glass Dounce homogeniser followed by centrifugation (500 g for 5 min at 4°C) and removal of the supernatant. The homgenisation step was subsequently repeated twice on the remaining cell pellet in order to maximise the yield of membranes. Supernantants were then pooled, further centrifuged (48,000 g for 60 min at 4°C) and finally resuspended in 1 mM sodium bicarbonate. Plasma membranes were further enriched using an aqueous extraction method (Morre, D.J. & Morre, D.M., 1989; BioTechniques 7; 9; 946-958).

Plasma membranes were incubated at 37°C in the presence and absence of inhibitor for 2 hours (Marolewski et al, 1998; Biochem. J.; 333; 573-579) following which time the reaction was stopped by the addition of 100 µM marimastat. Soluble CD23 shed from the plasma membranes was filtered through a 0.22 µm Millipore filter plate and quantitated by ELISA. IC₅₀ values were calculated by plotting inhibitor concentration versus % inhibition.

The functional effect of the compounds of the invention may be demonstrated using the following assays:

### Cellular CD23 Shedding Assay

The RPMI8866 cell line was routinely grown in RPMI1640 medium containing 10% FCS but was washed twice and resuspended in serum-free RPMI1640 medium immediately prior to the assay. Cells were then plated out in the presence and absence of inhibitor and incubated at 37°C in an atmosphere of 95% air/5% CO₂ for 1 hour (Christie et al., 1997; Eur. J. Immunol.; 27; 3228-3235). Following the time allocated, plates were centrifuged, the supernatants removed and subsequently analysed for shed soluble CD23 by ELISA. IC₅₀ values were calculated by plotting inhibitor concentration versus % inhibition.

### In Vitro Human IgE Synthesis

Mononuclear cells were isolated from human tonsillar tissue over a ficol gradient, washed in PBS and resuspended in RPMI1640 medium containing 10% FCS. Cells were then plated out, stimulated with 20 ng/ml IL-4/5 µg/ml anti-CD40 and incubated in the presence and absence of inhibitor at 37°C in an atmosphere of 95% air/5% CO₂ for 14 days (Christie et al., 1997; Eur. J. Immunol.; 27; 3228-3235). Following the time allocated, plates were centrifuged, the supernatants removed and subsequently analysed for human IgE by ELISA. IC₅₀ values were calculated by plotting inhibitor concentration versus % inhibition.

## Claims

1. A compound of formula (1): 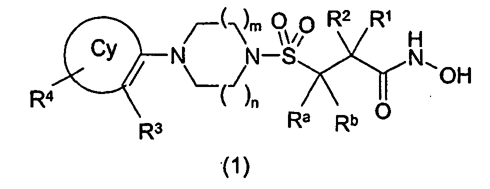 wherein:
Cy is an aryl or heteroaryl group;
m is zero or the integer 1, 2 or 3;
n is zero or the integer 1, 2 or 3; in which the sum of m and n is the integer 1, 2 or 3;
R¹ is a group selected from C₁₋₆alkyl, aryl, heteroaryl, heterocycloalkyl, C₃₋₆cycloalkyl, -C₁₋₆alkylaryl, -C₁₋₆alkylheteroaryl, -C₁₋₆alkylheterocycloalkyl or -C₁₋₆alkylC₃₋₆cycloalkyl, in which each aryl or heteroaryl group, present as or as part of the group R¹, may optionally be substituted with 1, 2 or 3 substituents selected from the group R⁷, wherein each R⁷ may be the same or different, and is an atom or group selected from F, Cl, Br, C₁₋₆alkyl, C₁₋₆haloalkyl, C₁₋₆alkoxy, C₁₋₆haloalkoxy, -CN, -CO₂R^{7a}, -CON(R^{7a})₂ or -COR^{7a}; and in which each alkyl, heterocycloalkyl or cycloalkyl group, present as or as part of the group R¹, may optionally be substituted with 1, 2 or 3 substituents selected from the group R⁸, wherein each R⁸ may be the same or different, and is an atom or group selected from F, C₁₋₆alkyl, C₁₋₆haloalkyl, C₁₋₆alkoxy, C₁₋₆haloalkoxy, =O, =NOR¹⁰, -CO₂R^{8a}, -CON(R^{8a})₂ or -COR^{8a};
R^{7a}, which may be the same or different, is each a hydrogen atom, or a C₁₋₆alkyl or C₁₋₆haloalkyl group;
R^{8a}, which may be the same or different, is each a hydrogen atom, or a C₁₋₆alkyl or C₁₋₆haloalkyl group;
R¹⁰ is a hydrogen atom or a C₁₋₃alkyl group;
R² is a hydrogen atom or a C₁₋₃alkyl group;
or R¹ and R² together with the carbon atom to which they are attached form a C₃₋₆cycloalkyl or heterocycloalkyl group optionally substituted with 1, 2 or 3 substituents selected from the group R⁹, wherein each R⁹ may be the same or different, and is an atom or group selected from F, C₁₋₆alkyl, C₁₋₆haloalkyl, C₁₋₆alkoxy, C₁₋₆haloalkoxy, =O, =NOR¹⁰, -CO₂R^{8a}, -CON(R^{8a})₂ or -COR^{8a};
R³ is an atom or group selected from F, Cl, Br, C₁₋₃alkyl, C₁₋₃haloalkyl, C₁₋₃alkoxy or C₁₋₃haloalkoxy;
R⁴ is a hydrogen, F, Cl or Br atom or a C₁₋₃alkyl, C₁₋₃haloalkyl, C₁₋₃alkoxy, C₁₋₃haloalkoxy, -CN, -SO₂R⁵, -SO₂N(R⁶)₂, -CON(R⁶)₂, -N(R⁶)₂, -NHSO₂R⁵ or -NHCOR⁵ group;
R⁵ is a C₁₋₃alkyl group;
R⁶, which may be the same or different, is each a hydrogen atom or a C₁₋₃alkyl group; and
R^{a} and R^{b}, which may be the same or different, is each an atom or group selected from hydrogen or C₁₋₃alkyl, or R^{a} and R^{b} may be joined to form a C₃₋₆cycloalkyl or heterocycloalkyl group as defined for R¹ and R²;
and the salts, solvates, hydrates, tautomers, isomers or *N*-oxides thereof.

2. A compound according to Claim 1 which has the formula (2): 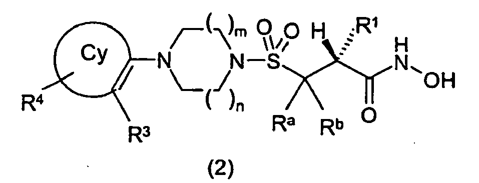 wherein m, n, Cy, R^{a}, R^{b}, R¹, R³ and R⁴ are as defined in Claim 1;
and the salts, solvates, hydrates, tautomers, isomers or *N*-oxides thereof.

3. A compound according to Claim 1 which has the formula (3): 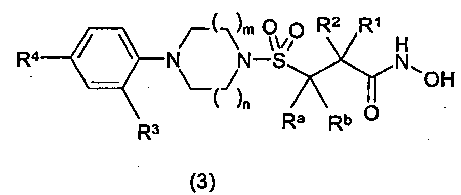 wherein m, n, R^{a}, R^{b}, R¹, R², R³ and R⁴ are as defined in Claim 1;
and the salts, solvates, hydrates, tautomers, isomers or *N*-oxides thereof.

4. A compound according to Claim 3 which has the formula (4): 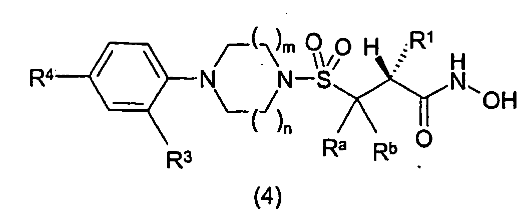 wherein m, n, R^{a}, R^{b}, R¹, R³ and R⁴ are as defined in Claim 1;
and the salts, solvates, hydrates, tautomers, isomers or *N*-oxides thereof.

5. A compound according to Claim 1 or Claim 2 wherein Cy is a phenyl group.

6. A compound according to any preceding Claim wherein R^{a} and R^{b} is each a hydrogen atom.

7. A compound according to any preceding Claim wherein m is the integer 1 and n is the integer 1.

8. A compound according to any preceding Claim in which R¹ is a group selected from C₁₋₆alkyl, phenyl, heteroaryl, heterocycloalkyl, C₃₋₆cycloalkyl, -(CH₂)₁₋₂phenyl, -(CH₂)₁₋₂heteroaryl, -(CH₂)₁₋₂heterocycloalkyl or -(CH₂)₁₋₂C₃₋₆cycloalkyl, in which each phenyl or heteroaryl group, present as or as part of the group R¹, may optionally be substituted with 1, 2 or 3 substituents selected from the group R⁷, as defined in Claim 1; and in which each alkyl, heterocycloalkyl or cycloalkyl group, present as or as part of the group R¹, may optionally be substituted with 1, 2 or 3 substituents selected from the group R⁸, as defined in Claim 1.

9. A compound according to any preceding Claim in which R¹ is a group selected from optionally substituted C₁₋₆alkyl, phenyl, heterocycloalkyl, C₃₋₆cycloalkyl or -(CH₂)₁₋₂phenyl.

10. A compound according to any one of Claims 1, 3 or 5 to 7 in which R¹ and R² together with the carbon atom to which they are attached form a C₃₋₆cycloalkyl group optionally substituted with 1, 2 or 3 substituents selected from the group R⁹, as defined in Claim 1.

11. A compound according to Claim 10 in which R¹ and R² together with the carbon atom to which they are attached form a cyclobutyl group.

12. A compound according to any preceding Claim in which R³ is an atom or group selected from F, Cl, methyl, ethyl, isopropyl, -CF₃, -CF₂H, methoxy, ethoxy, -OCF₃ or -OCF₂H.

13. A compound according to any preceding Claim in which R⁴ is an atom or group selected from hydrogen, F, Cl, methyl, -CF₃, methoxy, ethoxy, -OCF₃ or -OCF₂H.

14. A compound of any preceding Claim wherein R³ is an atom or group selected from F, Cl, methyl, ethyl or methoxy.

15. A compound according to Claim 14 wherein R³ is an atom or group selected from F, Cl, methyl or methoxy.

16. A compound according to Claim 14 wherein R³ is a methyl group.

17. A compound according to claim 1 which is:
*N*-hydroxy-3-methyl-2-(4-*o*-tolylpiperazine-1-sulfonylmethyl)butyramide;
*N*-hydroxy-3-methyl-2-[4-(2-methyl-4-fluorophenyl)piperazine-1-sulfonylmethyl]butyramide;
*N*-hydroxy-3-methyl-2-[4-(2,4-dimethylphenyl)piperazine-1-sulfonylmethyl]-butyramide;
*N*-hydroxy-3-methyl-2-[4-(2-methyl-4-trifluoromethoxyphenyl)piperazine-1-sulfonylmethyl]butyramide;
2-benzyl-*N*-hydroxy-3-(4-*o*-tolylpiperazine-1-sulfonyl)propionamide;
2-benzyl-*N*-hydroxy-3-[4-(2-methyl-4-trifluoromethoxyphenyl)piperazine-1-sulfonyl]propionamide;
*N*-hydroxy-2-phenyl-3-(4-*o*-tolylpiperazine-1-sulfonyl)propionamide;
*N*-hydroxy-2(*R*)-(tetrahydropyran-4-yl)-3-(4-*o*-tolylpiperazine-1-sulfonyl)-propionamide;
*N*-hydroxy-3-methyl-2(*R*)-(4-*o*-tolylpiperazine-1-sulfonylmethyl)butyramide;
1-(4-*o*-tolylpiperazine-1-sulfonylmethyl)cyclobutanecarboxylic acid hydroxyamide;
and the salts, solvates, hydrates, tautomers, isomers or *N*-oxides thereof.

18. A compound according to claim 1 which is:
*N*-hydroxy-3-methyl-2-[4-(2-methoxyphenyl)piperazine-1-sulfonylmethyl]-butyramide;
*N*-hydroxy-3-methyl-2-[4-(2-chlorophenyl)piperazine-1-sulfonylmethyl]-butyramide;
and the salts, solvates, hydrates, tautomers, isomers or *N*-oxides thereof.

19. A pharmaceutical composition comprising a compound according to any one of Claims 1 to 18 together with one or more pharmaceutically acceptable carriers, excipients or diluents.

## Patentansprüche

1. Verbindung der Formel (1): 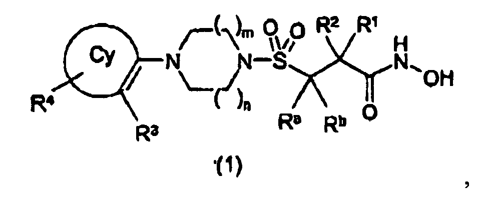 worin:
Cy eine Aryl- oder Heteroarylgruppe ist;
m null oder die ganze Zahl 1, 2 oder 3 ist;
n null oder die ganze Zahl 1, 2 oder 3 ist; wobei die Summe von m und n die ganze Zahl 1, 2 oder 3 ist;
R¹ eine Gruppe, ausgewählt aus C₁₋₆-Alkyl, Aryl, Heteroaryl, Heterocycloalkyl, C₃₋₆-Cycloalkyl, -C₁₋₆-Alkylaryl, -C₁₋₆-Alkylheteroaryl, -C₁₋₆-Alkylheterocycloalkyl oder -C₁₋₆-Alkyl-C₃₋₆-cycloalkyl ist, wobei jede Aryl- oder Heteroarylgruppe, die als die Gruppe R¹ oder ein Teil davon vorliegt, gegebenenfalls mit 1, 2 oder 3 Substituenten, ausgewählt aus der Gruppe R⁷, substituiert sein kann, wobei jeder R⁷ gleich oder verschieden sein kann und ein Atom oder eine Gruppe, ausgewählt aus F, Cl, Br, C₁₋₆-Alkyl, C₁₋₆-Halogenalkyl, C₁₋₆-Alkoxy, C₁₋₆-Halogenalkoxy, -CN, -CO₂R^{7a}, -CON(R^{7a})₂ oder -COR^{7a}, ist; und worin jede Alkyl-, Heterocycloalkyl- oder Cycloalkylgruppe, die als Gruppe R¹ oder ein Teil davon vorliegt, gegebenenfalls mit 1, 2 oder 3 Substituenten, ausgewählt aus der Gruppe R⁸, substituiert sein kann, worin jeder R⁸ gleich oder verschieden sein kann und ein Atom oder eine Gruppe, ausgewählt aus F, C₁₋₆-Alkyl, C₁₋₆-Halogenalkyl, C₁₋₆-Alkoxy, C₁₋₆-Halogenalkoxy, =O, =NOR¹⁰, -CO₂R^{8a}, -CON(R^{8a})₂ oder -COR^{8a}, ist;
R^{7a}, der gleich oder verschieden sein kann, jeweils ein Wasserstoffatom oder eine C₁₋₆-Alkyl- oder C₁₋₆-Halogenalkylgruppe ist;
R^{8a}, der gleich oder verschieden sein kann, jeweils ein Wasserstoffatom oder eine C₁₋₆-Alkyl- oder C₁₋₆-Halogenalkylgruppe ist;
R¹⁰ ein Wasserstoffatom oder eine C₁₋₃-Alkylgruppe ist;
R² ein Wasserstoffatom oder eine C₁₋₃-Alkylgruppe ist; oder
R¹ und R² zusammen mit dem Kohlenstoffatom, an das sie gebunden sind, eine C₃₋₆-Cycloalkyl- oder Heterocycloalkylgruppe bilden, die gegebenenfalls mit 1, 2 oder 3 Substituenten, ausgewählt aus der Gruppe R⁹, substituiert ist, worin jeder R⁹ gleich oder verschieden sein kann und ein Atom oder eine Gruppe, ausgewählt aus F, C₁₋₆-Alkyl, C₁₋₆-Halogenalkyl, C₁₋₆-Alkoxy, C₁₋₆-Halogenalkoxy, =O, =NOR¹⁰, -CO₂R^{8a}, -CON(R^{8a})₂ oder -COR^{8a}, ist;
R³ ein Atom oder eine Gruppe, ausgewählt aus F, Cl, Br, C₁₋₃-Alkyl, C₁₋₃-Halogenalkyl, C₁₋₃-Alkoxy oder C₁₋₃-Halogenalkoxy, ist;
R⁴ ein Wasserstoff-, F-, Cl- oder Br-Atom oder eine C₁₋₃-Alkyl-, C₁₋₃-Halogenalkyl-, C₁₋₃-Alkoxy-, C₁₋₃-Halogenalkoxy-, -CN-, -SO₂R⁵-, -SO₂N(R⁶)₂-, -CON(R⁶)₂-, -N(R⁶)₂-, -NHSO₂R⁵- oder -NHCOR⁵-Gruppe ist;
R⁵ eine C₁₋₃-Alkylgruppe ist;
R⁶, der gleich oder verschieden sein kann, jeweils ein Wasserstoffatom oder eine C₁₋₃-Alkylgruppe ist; und
R^{a} und R^{b}, die gleich oder verschieden sein können, jeweils ein Atom oder eine Gruppe, ausgewählt aus Wasserstoff oder C₁₋₃-Alkyl, sind, oder R^{a} und R^{b} unter Bildung einer C₃₋₆-Cycloalkyl- oder Heterocycloalkylgruppe, wie für R¹ und R² definiert, verbunden sein können;
und die Salze, Solvate, Hydrate, Tautomere, Isomere oder N-Oxide davon.

2. Verbindung nach Anspruch 1, die die Formel (2) aufweist: 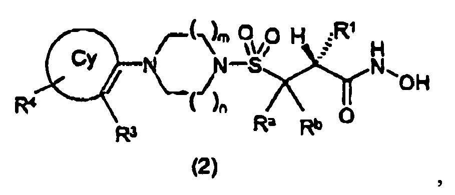 worin m, n, Cy, R^{a}, R^{b}, R¹, R³ und R⁴ wie in Anspruch 1 definiert sind;
und die Salze, Solvate, Hydrate, Tautomere, Isomere oder N-Oxide davon.

3. Verbindung nach Anspruch 1, die die Formel (3) aufweist: 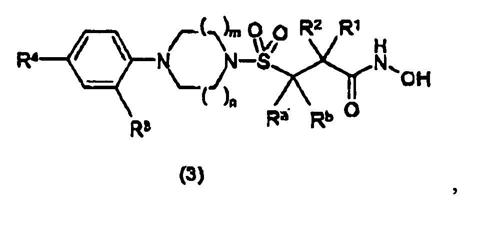 worin m, n, R^{a}, R^{b}, R¹, R², R³ und R⁴ wie in Anspruch 1 definiert sind;
und die Salze, Solvate, Hydrate, Tautomere, Isomere oder N-Oxide davon.

4. Verbindung nach Anspruch 3, die die Formel (4) aufweist: 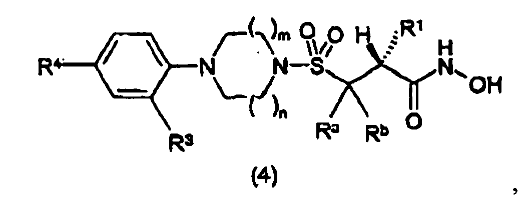 worin m, n, R^{a}, R^{b}, R¹, R³ und R⁴ wie in Anspruch 1 definiert sind;
und die Salze, Solvate, Hydrate, Tautomere, Isomere oder N-Oxide davon.

5. Verbindung nach Anspruch 1 oder Anspruch 2, worin Cy eine Phenylgruppe ist.

6. Verbindung nach einem vorhergehenden Anspruch, worin R^{a} und R^{b} jeweils ein Wasserstoffatom sind.

7. Verbindung nach einem vorhergehenden Anspruch, worin m die ganze Zahl 1 und n die ganze Zahl 1 ist.

8. Verbindung nach einem vorhergehenden Anspruch, worin R¹ eine Gruppe, ausgewählt aus C₁₋₆-Alkyl, Phenyl, Heteroaryl, Heterocycloalkyl, C₃₋₆-Cycloalkyl, -(CH₂)₁₋₂-Phenyl, -(CH₂)₁₋₂-Heteroaryl, -(CH₂)₁₋₂-Heterocycloalkyl oder -(CH₂)₁₋₂-C₃₋₆-Cycloalkyl ist, in der jede Phenyl- oder Heteroarylgruppe, die als Gruppe R¹ oder ein Teil dieser vorliegt, gegebenenfalls mit 1, 2 oder 3 Substituenten, ausgewählt aus der Gruppe R⁷, wie in Anspruch 1 definiert, substituiert sein kann; und in der jede Alkyl-, Heterocycloalkyl- oder Cycloalkylgruppe, die als Gruppe R¹ oder ein Teil dieser vorliegt, gegebenenfalls mit 1, 2 oder 3 Substituenten, ausgewählt aus der Gruppe R⁸, wie in Anspruch 1 definiert, substituiert sein kann.

9. Verbindung nach einem vorhergehenden Anspruch, worin R¹ eine Gruppe, ausgewählt aus gegebenenfalls substituiertem C₁₋₆-Alkyl, Phenyl, Heterocycloalkyl, C₃₋₆-Cycloalkyl oder -(CH₂)₁₋₂-Phenyl, ist.

10. Verbindung nach einem der Ansprüche 1, 3 oder 5 bis 7, worin R¹ und R² zusammen mit dem Kohlenstoffatom, an das sie gebunden sind, eine C₃₋₆-Cycloalkylgruppe bilden, die gegebenenfalls mit 1, 2 oder 3 Substituenten, ausgewählt aus der Gruppe R⁹, wie in Anspruch 1 definiert, substituiert ist.

11. Verbindung nach Anspruch 10, worin R¹ und R² zusammen mit dem Kohlenstoffatom, an das sie gebunden sind, eine Cyclobutylgruppe bilden.

12. Verbindung nach einem vorhergehenden Anspruch, worin R³ ein Atom oder eine Gruppe, ausgewählt aus F, Cl, Methyl, Ethyl, Isopropyl, -CF₃, -CF₂H, Methoxy, Ethoxy, -OCF₃ oder -OCF₂H, ist.

13. Verbindung nach einem vorhergehenden Anspruch, worin R⁴ ein Atom oder eine Gruppe, ausgewählt aus Wasserstoff, F, Cl, Methyl, -CF₃, Methoxy, Ethoxy, -OCF₃ oder -OCF₂H, ist.

14. Verbindung nach einem vorhergehenden Anspruch, worin R³ ein Atom oder eine Gruppe, ausgewählt aus F, Cl, Methyl, Ethyl oder Methoxy, ist.

15. Verbindung nach Anspruch 14, worin R³ ein Atom oder eine Gruppe, ausgewählt aus F, Cl, Methyl oder Methoxy, ist.

16. Verbindung nach Anspruch 14, worin R³ eine Methylgruppe ist.

17. Verbindung nach Anspruch 1, die:
N-Hydroxy-3-methyl-2-(4-o-tolylpiperazin-1-sulfonylmethyl)butyramid;
N-Hydroxy-3-methyl-2-[4-(2-methyl-4-fluorphenyl)piperazin-1-sulfonylmethyl]butyramid;
N-Hydroxy-3-methyl-2-[4-(2,4-dimethylphenyl)piperazin-1-sulfonylmethyl]-butyramid;
N-Hydroxy-3-methyl-2-[4-(2-methyl-4-trifluormethoxyphenyl)piperazin-1-sulfonylmethyl]-butyramid;
2-Benzyl-N-hydroxy-3-(4-o-tolylpiperazin-1-sulfonyl)propionamid;
2-Benzyl-N-hydroxy-3-[4-(2-methyl-4-trifluormethoxyphenyl)piperazin-1-sulfonyl]propionamid;
N-Hydroxy-2-phenyl-3-(4-o-tolylpiperazin-1-sulfonyl)propionamid;
N-Hydroxy-2(R)-(tetrahydropyran-4-yl)-3-(4-o-tolylpiperazin-1-sulfonyl)-propionamid;
N-Hydroxy-3-methyl-2(R)-(4-o-tolylpiperazin-1-sulfonylmethyl)butyramid;
1-(4-o-Tolylpiperazin-1-sulfonylmethyl)cyclobutancarbonsäure-hydroxyamid ist;
und die Salze, Solvate, Hydrate, Tautomere, Isomere oder N-Oxide davon.

18. Verbindung nach Anspruch 1, die:
N-Hydroxy-3-methyl-2-[4-(2-methoxyphenyl)piperazin-1-sulfonylmethyl]-butyramid;
N-Hydroxy-3-methyl-2-[4-(2-chlorphenyl)piperazin-1-sulfonylmethyl]-butyramid ist,
und die Salze, Solvate, Hydrate, Tautomere, Isomere oder N-Oxide davon.

19. Pharmazeutische Zusammensetzung, umfassend eine Verbindung nach einem der Ansprüche 1 bis 18 zusammen mit einem oder mehreren pharmazeutisch akzeptablen Trägem, Hilfsstoffen oder Verdünnungsmitteln.

## Revendications

1. Composé de formule ( 1 ) : 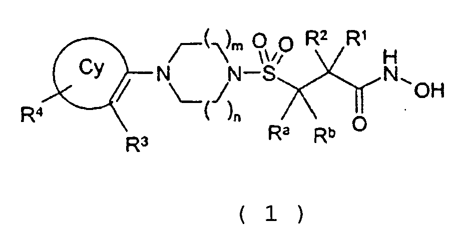 dans laquelle :
Cy est un groupe aryle ou hétéroaryle ;
m est zéro ou le nombre entier 1, 2 ou 3 ;
n est zéro ou le nombre entier 1, 2 ou 3 ; la somme de n et de m étant le nombre entier 1, 2 ou 3 ;
R¹ est un groupe choisi parmi alcoyle ayant de 1 à 6 atomes de carbone, aryle, hétéroaryle, hétérocycloalcoyle, cycloalcoyle ayant de 3 à 6 atomes de carbone, alcoylaryle ayant de 1 à 6 atomes de carbone dans la partie alcoyle, alcoylhétéroaryle ayant de 1 à 6 atomes de carbone dans la partie alcoyle, alcoylhétérocycloalcoyle ayant de 1 à 6 atomes de carbone dans la partie alcoyle ou alcoylcycloalcoyle ayant de 1 à 6 atomes de carbone dans la partie alcoyle et de 3 à 6 atomes de carbone dans la partie cycloalcoyle, dans lequel chaque groupe aryle ou hétéroaryle présent en tant que groupe R¹ ou en tant que partie du groupe R¹ peut être substitué éventuellement par 1, 2 ou 3 substituants choisis dans le groupe R⁷, chaque R⁷ pouvant être identique ou différent et étant un atome ou un groupe choisi parmi F, Cl, Br, alcoyle ayant de 1 à 6 atomes de carbone, haloalcoyle ayant de 1 à 6 atomes de carbone, alcoxy ayant de 1 à 6 atomes de carbone, haloalcoxy ayant de 1 à 6 atomes de carbone, CN, -CO₂R^{7a}, - CON(R^{7a})₂ ou -COR^{7a}; et dans lequel chaque groupe alcoyle, hétérocycloalcoyle ou cycloalcoyle présent en tant que groupe R¹ ou en tant que partie du groupe R¹ peut être substitué éventuellement par 1, 2 ou 3 substituants choisis dans le groupe R⁸, chaque R⁸ pouvant être identique ou différent et étant un atome ou un groupe choisi parmi F, alcoyle ayant de 1 à 6 atomes de carbone, haloalcoyle ayant de 1 à 6 atomes de carbone, alcoxy ayant de 1 à 6 atomes de carbone, haloalcoxy ayant de 1 à 6 atomes de carbone, =O, =NOR¹⁰, -CO₂R^{8a}, -CON(R^{8a})₂ ou -COR^{8a};
R^{7a}, qui peut être identique ou différent, est respectivement un atome d'hydrogène ou un groupe alcoyle ayant de 1 à 6 atomes de carbone ou haloalcoyle ayant de 1 à 6 atomes de carbone ;
R^{8a}, qui peut être identique ou différent, est respectivement un atome d'hydrogène ou un groupe alcoyle ayant de 1 à 6 atomes de carbone ou haloalcoyle ayant de 1 à 6 atomes de carbone ;
R¹⁰ est un atome d'hydrogène ou un groupe alcoyle ayant de 1 à 3 atomes de carbone ;
R² est un atome d'hydrogène ou un groupe alcoyle ayant de 1 à 3 atomes de carbone ;
ou R¹ et R² forment, ensemble avec l'atome de carbone auquel ils sont liés, un groupe cycloalcoyle ayant de 3 à 6 atomes de carbone ou hétérocycloalcoyle substitué éventuellement par 1, 2 ou 3 substituants choisis dans le groupe R⁹, chaque R⁹ pouvant être identique ou différent et étant un atome ou un groupe choisi parmi F, alcoyle ayant de 1 à 6 atomes de carbone, haloalcoyle ayant de 1 à 6 atomes de carbone, alcoxy ayant de 1 à 6 atomes de carbone, haloalcoxy ayant de 1 à 6 atomes de carbone, =O, =NOR¹⁰, -CO₂R^{8a}, -CON(R^{8a})₂ ou -COR^{8a};
R³ est un atome ou un groupe choisi parmi F, Cl, Br, alcoyle ayant de 1 à 3 atomes de carbone, haloalcoyle ayant de 1 à 3 atomes, alcoxy ayant de 1 à 3 atomes de carbone ou haloalcoxy ayant de 1 à 3 atomes de carbone ;
R⁴ est un atome d'hydrogène de F, de Cl ou de Br, ou un groupe alcoyle ayant de 1 à 3 atomes de carbone, haloalcoyle ayant de 1 à 3 atomes, alcoxy ayant de 1 à 3 atomes de carbone, haloalcoxy ayant de 1 à 3 atomes de carbone, -CN, - SO₂R⁵, -SO₂N(R⁶)₂, -CON(R⁶)₂, -N(R⁶)₂, -NHSO₂R⁵ ou -NHCOR⁵ ;
R⁵ est un groupe alcoyle ayant de 1 à 3 atomes de carbone ;
R⁶, qui peut être identique ou différent, est respectivement un atome d'hydrogène ou un groupe alcoyle ayant de 1 à 3 atomes de carbone ; et
R^{a} et R^{b}, qui peuvent être identiques ou différents, sont chacun un atome ou un groupe choisi parmi hydrogène ou alcoyle ayant de 1 à 3 atomes de carbone ou R^{a} et R^{b} peuvent être réunis pour former un groupe cycloalcoyle ayant de 3 à 6 atomes de carbone ou hétérocycloalcoyle tel que défini pour R¹ et R² ;
et leurs sels, produits de solvatation, hydrates, tautomères, isomères ou N-oxydes.

2. Composé suivant la revendication 1 qui a la formule ( 2 ) : 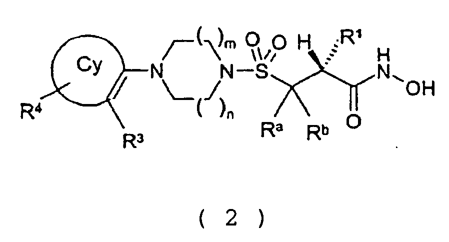 dans laquelle m, n, Cy, R^{a}, R^{b}, R¹, R³ et R⁴ sont tels que définis à la revendication 1 ;
et leurs sels, produits de solvatation, hydrates, tautomères, isomères ou N-oxydes.

3. Composé suivant la revendication 1 qui a la formule ( 3 ) : 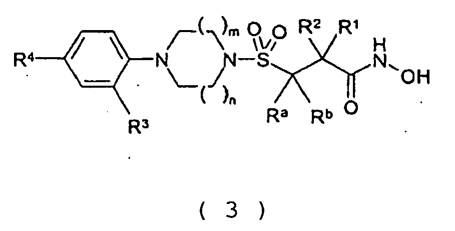 dans laquelle m, n, R^{a}, R^{b}, R¹, R², R³ et R⁴ sont tels que définis à la revendication 1 ;
et leurs sels, produits de solvatation, hydrates, tautomères, isomères ou N-oxydes.

4. Composé suivant la revendication 3 qui a la formule ( 4 ) : 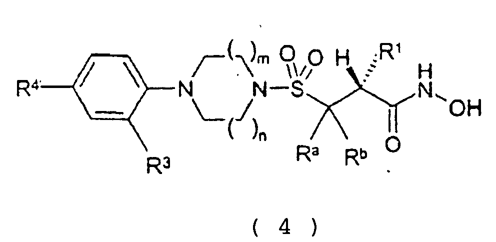 dans laquelle m, n, R^{a}, R^{b}, R¹, R³ et R⁴ sont tels que définis à la revendication 1 ;
et leurs sels, produits de solvatation, hydrates, tautomères, isomères ou N-oxydes.

5. Composé suivant la revendication 1 ou la revendication 2, dans lequel Cy est un groupe phényle.

6. Composé suivant l'une quelconque des revendications précédentes, dans lequel R^{a} et R^{b} sont chacun un atome d'hydrogène.

7. Composé suivant l'une quelconque des revendications précédentes, dans lequel m est le nombre entier 1 et n est le nombre entier 1.

8. Composé suivant l'une quelconque des revendications précédentes, dans lequel R¹ est un groupe choisi parmi alcoyle ayant de 1 à 6 atomes de carbone, phényle, hétéroaryle, hétérocycloalcoyle, cycloalcoyle ayant de 3 à 6 atomes de carbone, - (CH₂)_{1 à 2} phényle, - (CH₂)_{1 à 2} hétéroaryle, - (CH₂)_{1 à 2} hétérocycloalcoyle ou - (CH₂)_{1 à 2} cycloalcoyle ayant 3 à 6 atomes de carbone dans la partie cycloalcoyle, dans lequel chaque groupe phényle ou hétéroaryle présent en tant que groupe R¹ ou en tant que partie du groupe R¹ peut être substitué éventuellement par 1, 2 ou 3 substituants choisis dans le groupe R⁷ tel que défini à la revendication 1 ; et dans lequel chaque groupe alcoyle, hétérocycloalcoyle ou cycloalcoyle présent en tant que groupe R¹ ou en tant que partie du groupe du groupe R¹ peut être substitué éventuellement par 1, 2 ou 3 substituants choisis dans le groupe R⁸ tel que défini à la revendication 1.

9. Composé suivant l'une quelconque des revendications précédentes, dans lequel R¹ est un groupe choisi parmi alcoyle ayant de 1 à 6 atomes de carbone, phényle, hétérocycloalcoyle, cycloalcoyle ayant de 3 à 6 atomes de carbone ou -(CH₂)_{1 à 2} phényle éventuellement substitué.

10. Composé suivant l'une quelconque des revendications 1, 3 ou 5 à 7, dans lequel R¹ et R² forment ensemble avec l'atome de carbone auquel ils sont reliés un groupe cycloalcoyle ayant de 3 à 6 atomes de carbone substitué éventuellement par 1, 2 ou 3 substituants choisis dans le groupe R⁹ tel que défini à la revendication 1.

11. Composé suivant la revendication 10, dans lequel R¹ et R² forment ensemble avec l'atome de carbone auquel ils sont reliés un groupe cyclobutyle.

12. Composé suivant l'une quelconque des revendications précédentes, dans lequel R³ est un atome ou un groupe choisi parmi F, Cl, méthyle, éthyle, isopropyle, -CF₃, CF₂H, méthoxy, éthoxy, -OCF₃ ou -OCF₂H.

13. Composé suivant l'une quelconque des revendications précédentes, dans lequel R⁴ est un atome ou un groupe choisi parmi hydrogène, F, Cl, méthyle, -CF₃, méthoxy, éthoxy, -OCF₃ ou -OCF₂H.

14. Composé suivant l'une quelconque des revendications précédentes, dans lequel R³ est un atome ou un groupe choisi parmi F, Cl, méthyle, éthyle ou méthoxy.

15. Composé suivant la revendication 4, dans lequel R³ est un atome ou un groupe choisi parmi F, Cl, méthyle ou méthoxy.

16. Composé suivant la revendication 14, dans lequel R³ est un groupe méthyle.

17. Composé suivant la revendication 1 qui est :
le N-hydroxy-3-méthyl-2-(4-o-tolylpipérazine-1-sulfonylméthyl]butyramide ;
le N-hydroxy-3-méthyl-2-[4-(2-méthyl-4-fluorophényl)pipérazine-1-sulfonylméthyl]butyramide ;
le N-hydroxy-3-méthyl-2-[4-(2,4-diméthylphényl)pipérazine-1-sulfonylméthyl]butyramide ;
le N-hydroxy-3-méthyl-2-[4-(2-méthyl-4-trifluorométhoxyphényl)pipérazine-1-sulfonylméthyl]butyramide ;
le 2-benzyl-N-hydroxy-3-(4-o-tolylpipérazine-1-sulfonyl)propionamide ;
le 2-benzyl-N-hydroxy-3-[4-(2-méthyl-4-trifluorométhoxyphényl)pipérazine-1-sulfonylpropionamide ;
le N-hydroxy-2-phényl-3-(4-o-tolylpipérazine-1-sulfonyl]propionamide ;
le N-hydroxy-2(R)-(tétrahydropyran-4-yl)-3-(4-o-tolylpipérazine-1-sulfonyl)-propionamide ;
le N-hydroxy-3-méthyl-2(R)-(4-o-tolylpipérazine-1-sulfonylméthyl)butyramide ;
l'hydroxamide de l'acide 1-(4-o-tolylpipérazine-1-sulfonylméthyl)cyclobutanecarboxylique;
et leurs sels, produits de solvatation, hydrates, tautomères, isomères ou N-oxydes.

18. Composé suivant la revendication 1 qui est :
le N-hydroxy-3-méthyl-2-[4-(2-méthoxyphényl)pipérazine-1-sulfonylméthyl]butyramide ;
le N-hydroxy-3-méthyl-2-[4-(2-chlorophényl)pipérazine-1-sulfonylméthyl]butyramide ;
et leurs sels, produits de solvatation, hydrates, tautomères, isomères ou N-oxydes.

19. Composition pharmaceutique comprenant un composé suivant l'une quelconque des revendications 1 à 18, avec un ou plusieurs véhicules, excipients ou diluants acceptables pharmaceutiquement.
